(19) Europäisches Patentamt
European Patent Office
Office européen des brevets

(11) Veröffentlichungsnummer: **0 355 599 B1**

(12) # EUROPÄISCHE PATENTSCHRIFT

(45) Veröffentlichungstag der Patentschrift: **19.05.93**

(21) Anmeldenummer: **89114789.4**

(22) Anmeldetag: **10.08.89**

(51) Int. Cl.5: **C07D 471/04**, C07D 487/04, C07D 207/16, C07D 211/60, C07D 223/06, C07D 225/02, A01N 43/90, A61K 31/40, A61K 31/445, A61K 31/55, //(C07D471/04,221:00,209:00)

(54) **3-Aryl-pyrrolidin-2,4-dione.**

(30) Priorität: **20.08.88 DE 3828404**
**20.09.88 DE 3831852**
**26.04.89 DE 3913682**

(43) Veröffentlichungstag der Anmeldung:
**28.02.90 Patentblatt 90/09**

(45) Bekanntmachung des Hinweises auf die Patenterteilung:
**19.05.93 Patentblatt 93/20**

(84) Benannte Vertragsstaaten:
**BE CH DE ES FR GB GR IT LI NL**

(56) Entgegenhaltungen:
**EP-A- 0 262 399**
**DD-A- 259 993**
**US-A- 3 274 202**

(73) Patentinhaber: **BAYER AG**

**W-5090 Leverkusen 1 Bayerwerk(DE)**

(72) Erfinder: **Fischer, Reiner, Dr.**
**Nelly-Sachs-Strasse 23**
**W-4019 Monheim(DE)**
Erfinder: **Krebs, Andreas, Dr.**
**Im Gartenfeld 70**
**W-5068 Odenthal-Holz(DE)**
Erfinder: **Marhold, Albrecht, Dr.**
**Carl-Duisberg-Strasse 329**
**W-5090 Leverkusen 1(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**

EP 0 355 599 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 103, Nr. 5, 5. August 1985, Columbus, Ohio, US; R.SCHMIERER et al. "Cycliza- tion of N-acylalanine and N-acylglycine esters" Seite 538, Spalte 2, Zusammenfassung-Nr. 37 721g**

Erfinder: **Lürssen, Klaus, Dr.**
**August-Kierspel-Strasse 145**
**W-5060 Bergisch-Gladbach 2(DE)**
Erfinder: **Hagemann, Hermann, Dr.**
**Kandinsky-Strasse 52**
**W-5090 Leverkusen 1(DE)**
Erfinder: **Becker, Benedikt, Dr.**
**Metzkausener Strasse 14**
**W-4020 Mettmann(DE)**
Erfinder: **Schaller, Klaus, Dr.**
**Am Sonnenschein 38**
**W-5600 Wuppertal 1(DE)**
Erfinder: **Stendel, Wilhelm, Dr.**
**In den Birken 55**
**W-5600 Wuppertal 1(DE)**

## Beschreibung

Die Erfindung betrifft neue 3-Aryl-pyrrolidin-2,4-dion-(e)-Derivate, mehrere Verfahren zu ihrer Herstellung und ihre Verwendung als Herbizide, Fungizide, Antimykotika, Insektizide und Akarizide.

Von 3-Acyl-pyrrolidin-2,4-dionen sind pharmazeutische Eigenschaften vorbeschrieben (S. Suzuki et. al. Chem. Pharm. bull. 15 1120 (1967)). Weiterhin wurden N-Phenyl-pyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, fungizide, antimykotische, tickizide, insektizide oder akarizide Wirksamkeit bekannt geworden ist.

Es wurden nun neue 3-Aryl-pyrrolidin-2,4-dion-(e)-Derivate gefunden, die durch die Formel (I) dargestellt sind,

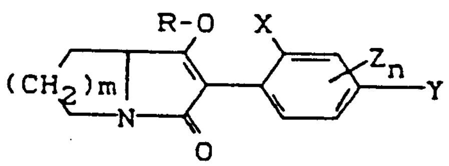

(I)

in welcher

X für $C_1-C_6$-Alkyl, Halogen, $C_1-C_6$-Alkoxy steht,

Y für Wasserstoff, $C_1-C_6$-Alkyl, Halogen, $C_1-C_6$-Alkoxy, $C_1-C_3$-Halogenalkyl steht,

Z für $C_1-C_6$-Alkyl, Halogen, $C_1-C_6$-Alkoxy steht,

m für eine Zahl von 1-4 steht,

n für eine Zahl von 0-3 steht,

R für Wasserstoff (Ia) oder für die Gruppen der Formel

$-CO-R^1$ (Ib),

$-CO-O-R^2$ (Ic)

oder

A (Id)

steht,

in welchen

A für ein Metallkationäquivalent oder für ein Ammoniumion steht,

$R^1$ für gegebenenfalls durch Halogen substituiertes: $C_1-C_{20}$-Alkyl, $C_2-C_{20}$-Alkenyl, $C_1-C_8$-Alkoxy-$C_1-C_8$-alkyl, $C_1-C_8$-Alkylthio-$C_1-C_8$-alkyl, $C_1-C_8$-Polyalkoxy-$C_2-C_8$-alkyl und Cycloalkyl mit 3-8 Ringatomen, das durch Sauerstoff und/oder Schwefel unterbrochen sein kann, steht,

für gegebenenfalls durch Halogen-, Nitro-, $C_1-C_6$-Alkyl-, $C_1-C_6$-Alkoxy-, $C_1-C_6$-Halogenalkyl-, $C_1-C_6$-Halogenalkoxy-substituiertes Phenyl;

für gegebenenfalls durch Halogen-, $C_1-C_6$-Alkyl, $C_1-C_6$-Alkoxy-, $C_1-C_6$-Halogenalkyl-, $C_1-C_6$-Halogenalkoxy-substituiertes Phenyl-$C_1-C_6$-alkyl steht,

für gegebenenfalls durch Halogen- und $C_1-C_6$-Alkyl-substituiertes Hetaryl steht,

für gegebenenfalls durch Halogen- und $C_1-C_6$-Alkyl-substituiertes Phenoxy-$C_1-C_6$-alkyl steht,

für gegebenenfalls durch Halogen, Amino und $C_1-C_6$-Alkyl-substituiertes Hetaryloxy-$C_1-C_6$-Alkyl steht,

$R^2$ für gegebenenfalls durch Halogen substituiertes: $C_1-C_{20}$-Alkyl, $C_2-C_{20}$-Alkenyl, $C_1-C_8$-Alkoxy-$C_2-C_8$-alkyl, $C_1-C_8$-Polyalkoxy-$C_2-C_8$-alkyl steht,

für gegebenenfalls durch Halogen-, $C_1-C_6$-Alkyl-, $C_1-C_6$-Alkoxy-, $C_1-C_6$-Halogenalkyl-substituiertes Phenyl steht,

sowie die enantiomerenreinen Formen von Verbindungen der Formel (I).

Im folgenden seien die folgenden Untergruppen definiert:

(Ia): Verbindungen der Formel (I) worin R = Wasserstoff,
(Ib): Verbindungen der Formel (I) worin R = $COR^1$,
(Ic): Verbindungen der Formel (I) worin R = $COOR^2$,
(Id): Verbindungen der Formel (I) worin R = ein Metallionäquivalent oder ein Ammoniumion.

(B)

Außerdem wurde gefunden, daß man Verbindungen der Formel (Ib)

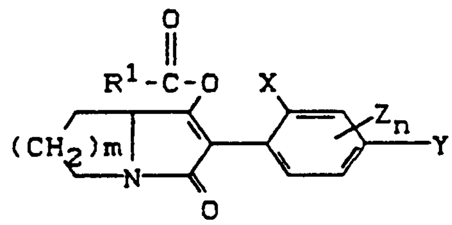

(Ib)

erhält, wenn man Verbindungen der Formel (Ia),

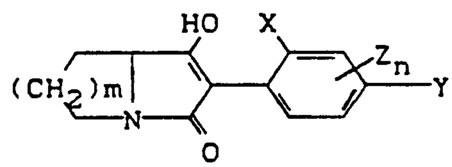

(Ia)

in welcher
X, Y, Z, m und n die oben angegebene Bedeutung haben,
α) mit Säurehalogeniden der allgemeinen Formel (III)

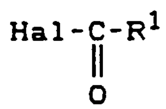

(III)

in welcher
$R^1$ die oben angegebene Bedeutung hat
und
Hal für Halogen, insbesondere Chlor und Brom steht,
gegebenenfalls in Gegenwart eines Verdünnungsmittels und gegebenenfalls in Gegenwart eines Säurebindemittels,
oder
β) mit Carbonsäureanhydriden der allgemeinen Formel (IV)

$R^1 - CO - O - CO - R^1$ (IV)

in welcher
$R^1$ die oben angegebene Bedeutung hat,
gegebenenfalls in Gegenwart eines Verdünnungsmittels und gegebenenfalls in Gegenwart eines Säurebindemittels,
umsetzt.

(C)

Ferner wurde gefunden, daß man Verbindungen der Formel (Ic)

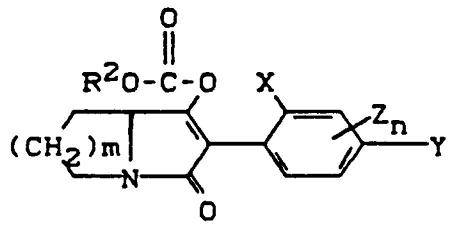 (Ic)

erhält, wenn man Verbindungen der Formel (Ia)

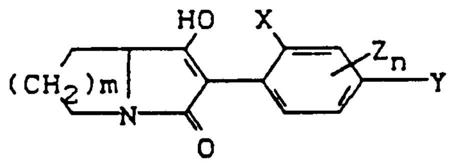 (Ia)

in welcher
X, Y, Z, m and n die oben angegebene Bedeutung haben
mit Chlorameisensäureester der allgemeinen Formel (V)

$R^2-O-CO-Cl$ (V)

in welcher
$R^2$ die oben angegebene Bedeutung hat,
gegebenenfalls in Gegenwart eines Verdünnungsmittels und gegebenenfalls in Gegenwart eines Säurebindemittels umsetzt.

(D)

Weiterhin wurde gefunden, daß man Verbindungen der Formel (Id)

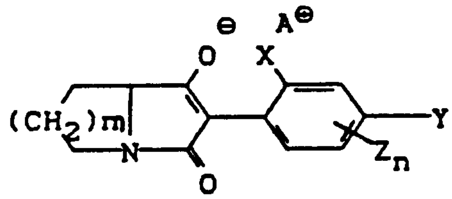 (Id)

in welcher X, Y, Z, A, m und n die oben angegebene Bedeutung haben,
erhält, wenn man Verbindungen der Formel (Ia)

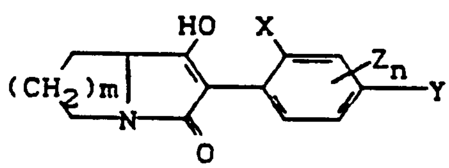 (Ia)

in welcher X, Y, Z, m und n die oben angegebene Bedeutung haben,
mit Metallhydroxiden oder Aminen der allgemeinen Formeln (VIII) und (IX)

$Me_sOH_t$ (VIII)

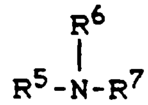 (IX)

in welchen
Me für ein- oder zweiwertige Metallionen
s und t für die Zahl 1 und 2 und
$R^5$, $R^6$ und $R^7$ unabhängig voneinander für Wasserstoff und Alkyl
stehen,
gegebenenfalls in Gegenwart eines Verdünnungsmittels, umsetzt.

Überraschenderweise wurde gefunden, daß die neuen 3-Arylpyrrolidin-2,4-dione der Formel (I) sich durch hervorragende herbizide, insektizide, antimykotische und akarizide Wirkungen auszeichnen.

Besonders bevorzugt sind Verbindungen der Formel (I) in welcher

X für $C_1-C_4$-Alkyl, Halogen, $C_1-C_4$-Alkoxy steht,
Y für Wasserstoff, $C_1-C_6$-Alkyl, Halogen, $C_1-C_4$-Alkoxy, $C_1-C_2$-Halogenalkyl steht,
Z für $C_1-C_4$-Alkyl, Halogen, $C_1-C_4$-Alkoxy steht,
m für eine Zahl von 1-3 steht,
n für eine Zahl von 0-3 steht,
R für Wasserstoff (Ia) oder für die Gruppen der Formel

-CO-$R^1$ (Ib) ,
-CO-O-$R^2$ (Ic)

oder

A (Id)

steht, in welchen
A für ein Metallkationäquivalent oder für ein Ammoniumion steht,
$R^1$ für gegebenenfalls durch Halogen substituiertes: $C_1-C_{20}$-Alkyl, $C_2-C_{20}$-Alkenyl, $C_1-C_8$-Alkoxy-$C_1-C_8$-alkyl, $C_1-C_8$-Alkylthio-$C_1-C_8$-alkyl, $C_1-C_8$-Polyalkoxy-$C_2-C_8$-alkyl und Cycloalkyl mit 3-8 Ringatomen, das durch Sauerstoff und/oder Schwefel unterbrochen sein kann, steht,
für gegebenenfalls durch Halogen-, Nitro-, $C_1-C_6$-Alkyl-, $C_1-C_6$-Alkoxy-, $C_1-C_6$-Halogenalkyl-, $C_1-C_6$-Halogenalkoxy-substituiertes Phenyl;
für gegebenenfalls durch Halogen-, $C_1-C_6$-Alkyl, $C_1-C_6$-Alkoxy-, $C_1-C_6$-Halogenalkyl-, $C_1-C_6$-Halogenalkoxy-substituiertes Phenyl-$C_1-C_6$-alkyl steht,
für gegebenenfalls durch Halogen- und $C_1-C_6$-Alkyl-substituiertes Hetaryl steht,
für gegebenenfalls durch Halogen- und $C_1-C_6$-Alkyl-substituiertes Phenoxy-$C_1-C_6$-alkyl steht,
für gegebenenfalls durch Halogen, Amino und $C_1-C_6$-Alkyl-substituiertes Hetaryloxy-$C_1-C_6$-Alkyl steht,
$R^2$ für gegebenenfalls durch Halogen substituiertes: $C_1-C_{20}$-Alkyl, $C_2-C_{20}$-Alkenyl, $C_1-C_8$-Alkoxy-$C_2-C_8$-alkyl, $C_1-C_8$-Polyalkoxy-$C_2-C_8$-alkyl steht,
für gegebenenfalls durch Halogen-, Nitro-, $C_1-C_6$-Alkyl-, $C_1-C_6$-Alkoxy-, $C_1-C_6$-Halogenalkyl-substituiertes Phenyl steht,

sowie die enantiomerenreinen Formen von Verbindungen der Formel (I).

Besonders bevorzugt sind Verbindungen der Formel (I) in welcher

X für $C_1-C_4$-Alkyl, Halogen, $C_1-C_4$-Alkoxy steht,
Y für Wasserstoff, $C_1-C_6$-Alkyl, Halogen, $C_1-C_4$-Alkoxy, $C_1-C_2$-Halogenalkyl steht,
Z für $C_1-C_4$-Alkyl, Halogen, $C_1-C_4$-Alkoxy steht,
m für eine Zahl von 1-3 steht,
n für eine Zahl von 0-3 steht,
R für Wasserstoff (Ia) oder für die Gruppen der Formel

-CO-$R^1$ (Ib) ,

$-CO-O-R^2$ (Ic)

oder

A (Id)

steht, in welchen

A für ein Metallkationäquivalent oder für ein Ammoniumion steht,

$R^1$ für gegebenenfalls durch Halogen substituiertes: $C_1-C_{16}$-Alkyl, $C_2-C_{16}$-Alkenyl, $C_1-C_6$-Alkoxy-$C_1-C_6$-alkyl, $C_1-C_6$-Alkylthio-$C_1-C_6$-alkyl, $C_1-C_6$-Polyalkoxy-$C_2-C_6$-alkyl und Cycloalkyl mit 3-7 Ringatomen, das durch 1-2 Sauerstoff- und/oder Schwefelatome unterbrochen sein kann steht,
für gegebenenfalls durch Halogen-, Nitro-, $C_1-C_4$-Alkyl-, $C_1-C_4$-Alkoxy-, $C_1-C_3$-Halogenalkyl-, $C_1-C_3$-Halogenalkoxy-substituiertes Phenyl steht,
für gegebenenfalls durch Halogen-, $C_1-C_4$-Alkyl-, $C_1-C_4$-Alkoxy-, $C_1-C_3$-Halogenalkyl-, $C_1-C_3$-Halogenalkoxy-substituiertes Phenyl-$C_1-C_4$-alkyl steht,
für gegebenenfalls duch Halogen- und $C_1-C_6$-Alkyl-substituiertes Hetaryl steht,
gegebenenfalls für durch Halogen- und $C_1-C_4$-Alkyl-substituiertes Phenoxy-$C_1-C_5$-alkyl steht,
für gegebenenfalls durch Halogen, Amino und $C_1-C_4$-Alkyl-substituiertes Hetaryloxy-$C_1-C_5$-alkyl steht,

$R^2$ gegebenenfalls durch Halogen substituiertes: $C_1-C_{16}$-Alkyl, $C_2-C_{16}$-Alkenyl, $C_1-C_{16}$-Alkoxy-$C_2-C_6$-alkyl, $C_1-C_6$-Polyalkoxy-$C_2-C_6$-alkyl steht,
für gegebenenfalls durch Halogen, Nitro-, $C_1-C_4$-Alkyl, $C_1-C_3$-Alkoxy-, $C_1-C_3$-Halogenalkyl-substituiertes Phenyl steht,

sowie die enantiomerenreinen Formen von Verbindungen der Formel (I).

Ganz besonders bevorzugt sind Verbindungen der Formel (I) in welcher

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Chlor, Brom, Methoxy, Ethoxy und Trifluormethyl steht,

Z für Methyl, Ethyl, i-Propyl, Butyl, i-Butyl, tert.-Butyl, Chlor, Brom, Methoxy und Ethoxy steht,

m für eine Zahl von 1-2 steht,

n für eine Zahl von 0-3 steht,

R für Wasserstoff (Ia) oder für die Gruppen der Formel

$-CO-R^1$ (Ib) ,
$-CO-O-R^2$ (Ic)

oder

A (Id)

steht, in welcher

A für ein Metallkationäquivalent oder für ein Ammoniumion steht,

$R^1$ für gegebenenfalls durch Fluor oder Chlor substituiertes: $C_1-C_{14}$-Alkyl, $C_2-C_{14}$-Alkenyl, $C_1-C_4$-Alkoxy-$C_1-C_6$-alkyl, $C_1-C_4$-Alkylthio-$C_1-C_6$-alkyl, $C_1-C_4$-Polyalkoxyl-$C_2-C_4$-alkyl und Cycloalkyl mit 3-6 Ringatomen, das durch 1-2 Sauerstoff- und/oder Schwefelatome unterbrochen sein kann steht,
für gegebenenfalls durch Fluor-, Chlor, Brom-, Methyl-, Ethyl-, Propyl, i-Propyl, Methoxy, Ethoxy, Trifluormethyl-, Trifluormethoxy-, Nitro- substituiertes Phenyl steht,
für gegebenenfalls durch Fluor-, Chlor-, Brom-, Methyl-, Ethyl-, Propyl-, i-Propyl, Methoxy, Ethoxy, Trifluormethyl, Trifluormethoxy- substituiertes Phenyl-$C_1-C_3$-alkyl steht,
für gegebenenfalls durch Fluor-, Chlor-, Brom-, Methyl-, Ethyl-substituiertes Pyridyl, Pyrimidyl, Thiazolyl und Pyrazolyl steht,
für gegebenenfalls durch Fluor-, Chlor-, Methyl-, Ethyl-substituiertes Phenoxy-$C_1-C_4$-alkylsteht,
für gegebenenfalls durch Fluor-, Chlor-, Amino-, Methyl-, Ethyl-, substituiertes Pyridyloxy-$C_1-C_4$-alkyl, Pyrimidyloxy-$C_1-C_4$-alkyl und Thiazolyloxy-$C_1-C_4$-alkyl steht,

$R^2$ für gegebenenfalls durch Fluor oder Chlor substituiertes: $C_1-C_{14}$-Alkyl, $C_2-C_{14}$-Alkenyl, $C_1-C_4$-Alkoxy-$C_2-C_6$-alkyl, $C_1-C_4$-Polyalkoxy-$C_2-C_6$-alkyl steht,
für gegebenenfalls durch Fluor-, Chlor-, Nitro-, Methyl-, Ethyl-, Propyl-, i-Propyl-, Methoxy-, Ethoxy-, Trifluormethyl-, substituiertes Phenyl steht,

sowie die enantiomerenreinen Formen von Verbindungen der Formel I.

Verwendet man gemäß Verfahren (A) N-(2,6-Dichlorphenylacetyl)-piperidin-2-carbonsäureethylester, so kann der Verlauf des erfindungsgemäßen Verfahrens durch folgendes Reaktionsschema wiedergegeben werden:

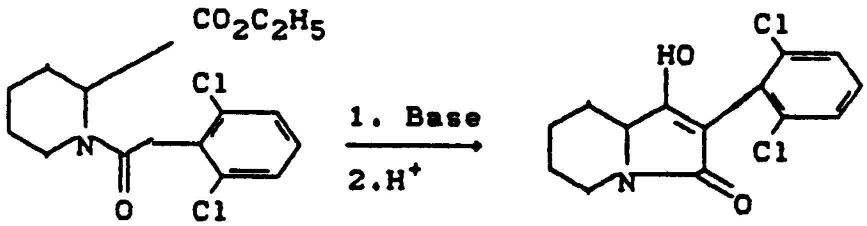


Verwendet man gemäß Verfahren (B) (Variante α) 3-(2,4,6-Trimethylphenyl)-1,5-trimethylen-pyrrolidin-2,4-dion und Pivaloylchlorid als Ausgangsstoff, so kann der Verlauf des erfindungsgemäßen Verfahrens durch folgendes Reaktionsschema wiedergegeben werden.

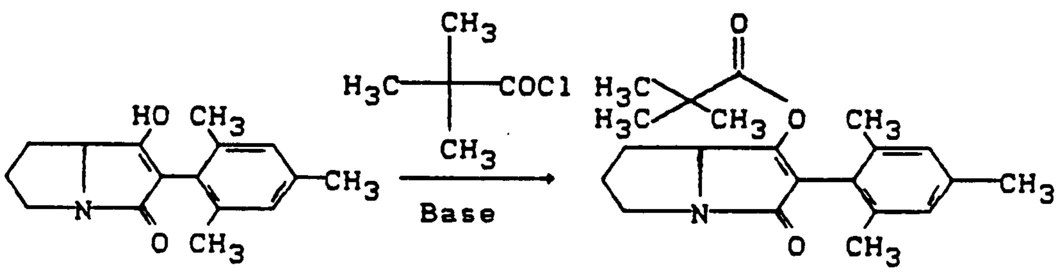


Verwendet man gemäß Verfahren B (Variante β) 3-(2,4,5-Trimethylphenyl)-1,5-tetramethylen-pyrrolidin-2,4-dion und Acetanhydrid, so kann der Verlauf des erfindungsgemäßen Verfahrens durch folgendes Reaktionsschema wiedergegeben werden.

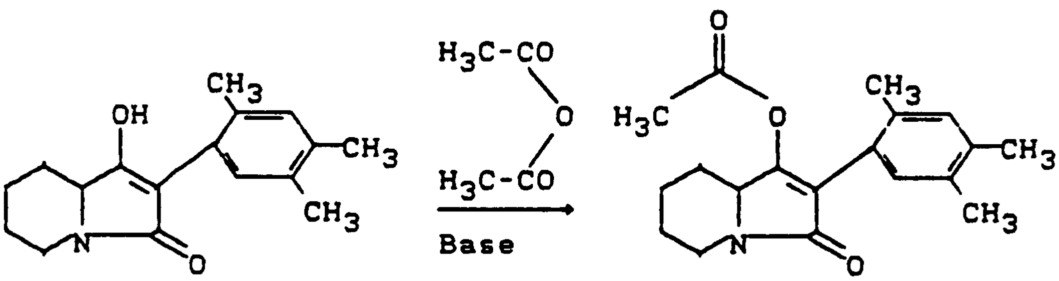


Verwendet man gemäß Verfahren C 3-(2,4-Dichlorphenyl)-1,5-tetramethylen-pyrrolidin-2,4-dion und Chlorameisensäureethoxyethylester, so kann der Verlauf des erfindungsgemäßen Verfahrens durch folgendes Reaktionsschema wiedergegeben werden.

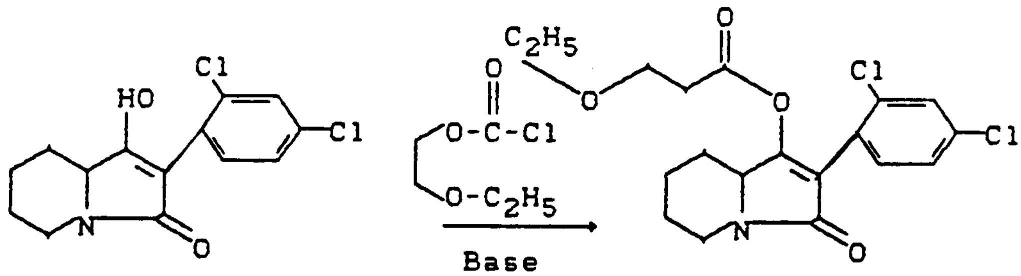


Verwendet man gemäß Verfahren (D) 3-(2,4,6-Trimethylphenyl)-1,5-trimethylen-pyrrolidin-2,4-dion und NaOH, so kann der Verlauf des erfindungsgemäßen Verfahrens durch folgendes Reaktionsschema wiedergegeben werden:

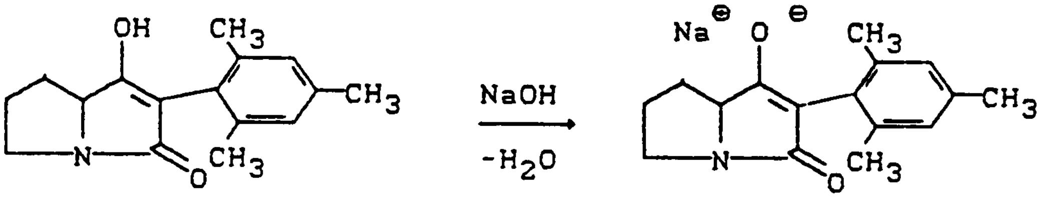


Die bei dem obigen Verfahren (A) als Ausgangsstoffe benötigten Verbindungen der Formel (II)

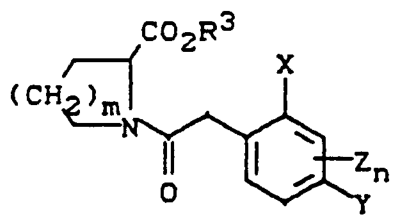

(II)

in welcher

X, Y, Z, m, n und $R^3$ die oben angegebene Bedeutung haben sind nicht bekannt, lassen sich aber nach im Prinzip bekannten Methoden in einfacher Weise herstellen. So erhält man z.B. Acyl-aminosäureester der Formel (II), wenn man

a) Aminosäureester der Formel (VI),

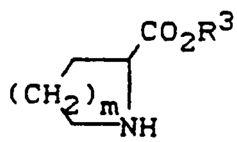

(VI)

in welcher

$R^3$ für Alkyl

und

m für die Zahl 1 oder 2 steht,

mit Phenylessigsäurehalogeniden der Formel (VII)

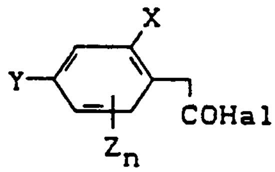

(VII)

in welcher
X, Y, Z und n die oben angegebene Bedeutung haben
und
Hal für Chlor oder Brom steht,
acyliert (Allgemeine Methodik beschrieben in: Chem. Reviews 52 237 – 416 (1953));
oder wenn man
b) Acylaminosäuren der Formel (IIa),

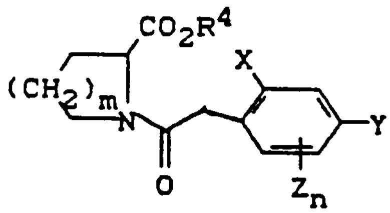

(IIa)

in welcher
X, Y, Z, m und n die oben angegebene Bedeutung haben
und
$R^4$ für Wasserstoff steht,
verestert (Allgemeine Methodik beschrieben in: Chem. Ind. (London) 1568 (1968)).

Verbindungen der Formel (IIa) sind beispielsweise aus den Phenylessigsäurehalogeniden der Formel (VII) und Aminosäuren der Formel (VIa)

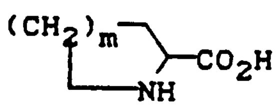

(VIa)

in welcher
m für die Zahl 1 oder 2 steht,
nach Schotten – Baumann (Organikum 9. Auflage 446 (1970) VEB Deutscher Verlag der Wissenschaften, Berlin) erhältlich.

Beispielhaft sind folgende Verbindungen der Formel (II) genannt:
1) N – (2,4 – Dichlorphenylacetyl) – piperidin – 2 – carbonsäureethylester
2) N – (2 – Fluor – 4 – chlorphenylacetyl) – piperidin – 2 – carbonsäurethylester
3) N – (2,6 – Dichlorphenylacetyl) – piperidin – 2 – carbonsäureethylester
4) N – (2 – Fluor – 6 – chlorphenylacetyl) – piperidin – 2 – carbonsäureethylester
5) N – (2,4,6 – Trimethylphenylacetyl) – piperidin – 2 – carbonsäureethylester
6) N – (2 – Fluor – 6 – chlor – 4 – trifluormethylphenylacetyl) – piperidin – 2 – carbonsäureethylester
7) N – (2,6 – Dichlor – 4 – trifluormethylphenylacetyl) – piperidin – 2 – carbonsäureethylester
8) N – (2,4,5 – Trimethyl – phenylacetyl) – piperidin – 2 – carbonsäureethylester
9) N – (2 – Fluor – 5 – chlor – 4 – trifluormethylphenylacetyl) – piperidin – 2 – carbonsäureethylester
10) N – (2,4,6 – Triisopropyl – phenylacetyl) – piperidin – 2 – carbonsäureethylester
11) N – (2,4,6 – Trichlorphenylacetyl) – piperidin – 2 – carbonsäureethylester
12) N – (2 – Chlor – 3 – methyl – phenylacetyl) – piperidin – 2 – carbonsäureethylester
13) N – (3 – Brom – 2,4,6 – trimethyl – phenylacetyl) – piperidin – 2 – carbonsäureethylester
14) N – (Pentamethyl – phenylacetyl) – piperidin – 2 – carbonsäureethylester
15) N – (4 – tert. – Butyl – 2 – methyl – phenylacetyl) – piperidin – 2 – carbonsäureethylester

16) N – (4 – tert. – Butyl – 2,6 – dimethyl – phenylacetyl) – piperidin – 2 – carbonsäureethylester
17) N – (2,3,4,6 – Tetramethyl – phenylacetyl) – piperidin – 2 – carbonsäureethylester
18) N – (2,3,6 – Trichlor – phenylacetyl) – piperidin – 2 – carbonsäureethylester
19) N – (2,4 – Dimethyl – phenylacetyl) – piperidin – 2 – carbonsäureethylester
20) N – (2,3,4,5 – Tetramethyl – phenylacetyl) – piperidin – 2 – carbonsäureethylester
21) N – (2,3,5,6 – Tetramethyl – phenylacetyl) – piperidin – 2 – carbonsäureethylester
22) N – (2 – Fluor – 4,6 – dimethyl – phenylacetyl) – piperidin – 2 – carbonsäureethylester
23) N – (4 – Fluor – 2,6 – dimethyl – phenylacetyl) – piperidin – 2 – carbonsäureethylester
24) N – (2,4 – Dichlor – phenylacetyl) – pyrrolidin – 2 – carbonsäuremethylester
25) N – (2,6 – Dichlor – phenylacetyl) – pyrrolidin – 2 – carbonsäuremethylester
26) N – (2,4,6 – Trimethyl – phenylacetyl) – pyrrolidin – 2 – carbonsäuremethylester
27) N – (2,4,5 – Trimethyl – phenylacetyl) – pyrrolidin – 2 – carbonsäuremethylester
28) N – (2 – Fluor – 6 – chlor – phenylacetyl) – pyrrolidin – 2 – carbonsäuremethylester
29) N – (2,6 – Dichlor – 4 – trifluormethyl – phenylacetyl) – pyrrolidin – 2 – carbonsäuremethylester
30) N – (2,4,6 – Trichlor – phenylacetyl) – pyrrolidin – 2 – carbonsäuremethylester
31) N – (2,3,6 – Trichlor – phenylacetyl) – pyrrolidin – 2 – carbonsäuremethylester
32) N – (2 – Fluor – 4,6 – dimethyl – phenylacetyl) – pyrrolidin – 2 – carbonsäuremethylester
33) N – (4 – Fluor – 2,6 – dimethyl – phenylacetyl) – pyrrolidin – 2 – carbonsäuremethylester

Beispielhaft seien folgende Verbindungen der Formel (IIa) genannt:

34) N – 2,4 – Dichlorphenylacetyl – prolin
35) N – 2 – Fluor – 6 – chlorphenylacetyl – prolin
36) N – 2,6 – Dichlorphenylacetyl – prolin
37) N – 2,4,6 – Trimethylphenylacetyl – prolin
38) N – 2,4,5 – Trimethylphenylacetyl – prolin
39) N – 2,6 – Dichlor – 4 – trifluormethyl – phenylacetyl – prolin

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0 ° C und 250 ° C, vorzugsweise zwischen 50 ° C und 150 ° C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (A) ist dadurch gekennzeichnet, daß Verbindungen der Formel (II) in welcher X, Y, Z, m, n und $R^3$ die obenangegebene Bedeutung haben in Gegenwart von Basen einer intramolekularen Kondensation unterwirft.

Als Verdünnungsmittel können bei dem erfindungsgemäßen Verfahren (A) alle üblichen inerten organischen Solventien eingesetzt werden. Vorzugsweise verwendbar sind Kohlenwasserstoffe, wie Cyclohexan, Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N – Methyl – pyrrolidon.

Als Deprotonierungsmittel können bei der Durchführung des erfindungsgemäßen Verfahrens (A) alle üblichen Protonenakzeptoren eingesetzt werden. Vorzugsweise verwendbar sind Alkalimetall – und Erdalkalimetall – oxide, – hydroxide und – carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind Alkalimetall – und Erdalkalimetallamide und – hydride, wie Natriumamid, Natriumhydrid und Calciumhydrid, und außerdem auch Alkalimetall – alkoholate, wie Natrium – methylat, Natriummethylat und Kalium – tert. – butylat einsetzbar.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise

Adogen 464 = Methyltrialkyl($C_8$-$C_{10}$)ammoniumchlorid

TDA 1 = Tris-(methoxyethoxylethyl)-amin

verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzten. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können auch bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern der Formel (V) umsetzt.

Verwendet man die entsprechenden Chlorameisensäureester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende (Chlorameisensäureester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Das Verfahren (D) ist dadurch gekennzeichnet, daß man Verbindungen der Formel (Ia) mit Acetalhydroxiden (VIII) oder Aminen(IX) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (D) wird im allgemeinen unter Normaldruck durchgeführt. Die Reaktionstemperaturen liegen im allgemeinen zwischen −20 °C und 100 °C, vorzugsweise zwischen 0 °C und 50 °C.

Bei der Durchführung des erfindungsgemäßen Verfahrens (D) werden die Ausgangsstoffe der Formel (Ia) bzw. (IX) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittels einengt.

Beispiel 1:

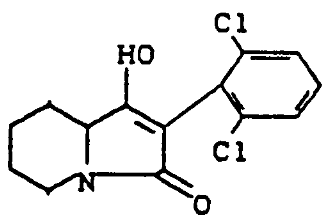

8,4 g (0,28 Mol) Natriumhydrid (80%ig) werden in 150 ml abs. Toluol vorgelegt. Nach Zutropfen von 80 g (0,23 Mol) N-(2,6-Dichlorphenylacetyl)-piperidin-2-carbonsäureethylester in 400 ml abs. Toluol, erhitzt man 6 h unter Rückfluß. Unter Eisbadkühlung wurden 30 ml Ethanol zugetropft, der Ansatz im Vakuum einrotiert, der Rückstand in 1 N NaOH gelöst und das 3-(2,6-Dichlorphenyl)-1,5-tetramethylen-pyrrolidin-2,4-dion bei 0-20 °C mit konzentrierter HCl gefällt. Das Produkt wird zur Reinigung mit Chloroform ausgekocht, anschließend wird n-Hexan zugesetzt und das ausgefallene, farblose Produkt abgesaugt.
Ausbeute: 40,5 g (59,1% d. Theorie) Fp. > 250 °C.

Beispiel 2:

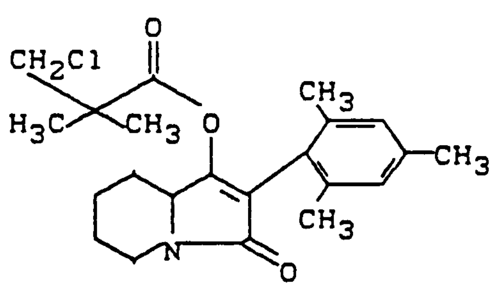

4,6 g (15 mmol) 3-(2,4,6-Trimethylphenyl)-1,5-tetramethylen-pyrrolidin-2,4-dion werden in 50 ml abs. THF suspendiert und mit 1,22 ml (15 mmol) abs. Pyridin und 2,54 ml (15 mmol) Ethyldiisopropylamin versetzt. Dazu tropft man bei 0° -10 °C 1,94 ml (15 mmol) 3-Chlorpivaloylchlorid gelöst in 5 ml abs. THF und rührt 30 Min. nach. Der Niederschlag wird abfiltriert, die Lösung im Vakuum einrotiert und der Rückstand an Kieselgel mit Cyclohexan/Essigester 1:1 chromatographiert.

Durch Kristallisation aus Ether/n – Hexan erhält man 3,93 g (70% der Theorie) 4 – (3 – Chlorpivaloy – loxy) – 3 – (2,4,6 – trimethylphenyl) – 1,5 – tetramethylen – 3 – pyrrolin – 2 – on von Schmp. 102 ° C.

Beispiel 3

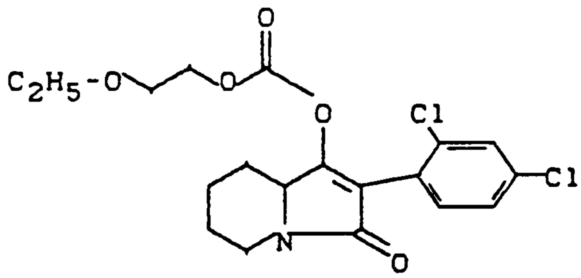

4,47 g (15 mmol) 3 – (2,4 – Dichlorphenyl) – 1,5 – tetramethylenpyrrolidin – 2,4 – dion werden in 50 ml abs. THF mit 1,22 ml (15 mmol) abs. Pyridin versetzt. Bei 0 ° C – 10 ° C werden 2,44 g (15 mmol) Chlorameisensäureethoxyethylester gelöst in 5 ml abs. THF zugetropft und 30 Min. nachgerührt. Nach Abfiltrieren des Niederschlages wird das Filtrat im Vakuum einrotiert, der Rückstand an Kieselgel mit Cyclohexan/Essigester 1:2 chromatographiert und aus Ether/n – Hexan kristallisiert.
Ausbeute: 5,2 g (83,7% der Theorie) 4 – Ethoxyethyl – oxycarbonyloxy – 3 – (2,4 – dichlorphenyl) – 1,5 – tetramethylen – 3 – pyrrolin – 2 – on vom Schmp. 80 ° C.

In entsprechender Weise zu den Herstellungsbeispielen und gemäß den allgemeinen Angaben zur Herstellung erhält man die in den folgenden Tabellen 1 – 3 formelmäßig aufgeführten 3 – Aryl – pyrrolidin – 2,4 – dion(e) – Derivate der Formel (Ia) – (Ic).

**Tabelle 1**

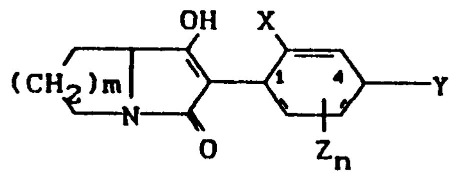

(Ia)

| Bsp. Nr. | X | Y | $Z_n$ | m | Fp. °C |
|---|---|---|---|---|---|
| 4 | Cl | Cl | - | 1 | ›218 |
| 5 | Cl | H | 6-Cl | 1 | ›230 |
| 6 | $CH_3$ | $CH_3$ | 5-$CH_3$ | 1 | 228 |
| 7 | $CH_3$ | $CH_3$ | 6-$CH_3$ | 1 | ›230 |
| 8 | Cl | H | - | 2 | 174 |
| 9 | F | Cl | - | 2 | 207 |
| 10 | Cl | Cl | - | 2 | 208 |
| 11 | Cl | H | 6-F | 2 | 230 |
| 12 | $CH_3$ | $CH_3$ | 5-$CH_3$ | 2 | 210 |
| 13 | $CH_3$ | $CH_3$ | 6-$CH_3$ | 2 | ›230 |
| 14 | F | $CF_3$ | 5-F | 2 | 228 |
| 15 | F | $CF_3$ | 5-Cl | 2 | ›230 |
| 16 | F | $CF_3$ | 6-Cl | 2 | 227 |
| 17 | Cl | $CF_3$ | 6-Cl | 2 | ›230 |
| 18 | $CH_3$ | H | 6-$CH_3$ | 2 | |
| 19 | $CH_3$ | H | 6-Cl | 2 | |

Tabelle 1 (Fortsetzung)

| Bsp. Nr. | X | Y | $Z_n$ | m | Fp. °C |
|---|---|---|---|---|---|
| 20 | $CH_3$ | $CH_3$ | 3-$CH_3$ | 2 | |
| 21 | $CH_3$ | $CH_3$ | 3.5-di-$CH_3$ | 2 | 160 |
| 22 | $CH_3$ | $CH_3$ | 3.5.6-tri-$CH_3$ | 2 | >230 |
| 23 | $CH_3$ | t-$C_4H_9$ | 6-$CH_3$ | 2 | >230 |
| 24 | $CH_3$ | F | - | 2 | 191 |
| 25 | $CH_3$ | $CH_3$ | - | 2 | 192 |
| 26 | $CH_3$ | t-$C_4H_9$ | - | 2 | 210 |
| 27 | Cl | H | 3-$CH_3$ | 2 | 195 |
| 28 | Cl | H | 3.5-di-Cl | 2 | >230 |
| 29 | Cl | Cl | 6-Cl | 2 | >230 |
| 30 | $CH_3$ | F | 6-$CH_3$ | 2 | >230 |
| 31 | $CH_3$ | $CH_3$ | 6-F | 2 | 207 |
| 32 | i-$C_3H_7$ | i-$C_3H_7$ | 6-i-$C_3H_7$ | 2 | >230 |
| 33 | $CH_3$ | H | 3.5.6-tri-$CH_3$ | 2 | >230 |
| 34 | $CH_3$ | $CH_3$ | 3.6-di-$CH_3$ | 2 | >230 |
| 35 | $CH_3$ | $CH_3$ | 3-Br-6-$CH_3$ | 2 | >230 |

Tabelle 2

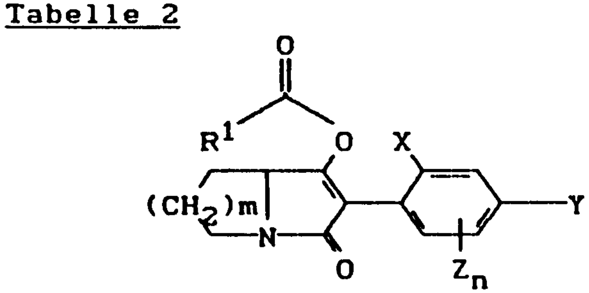

(Ib)

| Bsp. Nr. | X | Y | $Z_n$ | m | $R^1$ | Fp. °C |
|---|---|---|---|---|---|---|
| 36 | Cl | Cl | - | 1 | $CH_3-$ | 95 |
| 37 | Cl | Cl | - | 1 | $(CH_3)_2CH-$ | Öl |
| 38 | Cl | Cl | - | 1 | $(CH_3)_3C-$ | Öl |
| 39 | Cl | Cl | - | 1 | $(CH_3)_2-CH-C(CH_3)_2-$ | Öl |
| 40 | Cl | H | 6-Cl | 1 | $CH_3-$ | 80 |
| 41 | Cl | H | 6-Cl | 1 | $(CH_3)_2CH-$ | |
| 42 | Cl | H | 6-Cl | 1 | $(CH_3)_3C-$ | 102 |
| 43 | Cl | H | 6-Cl | 1 | $(CH_3)_2CH-C(CH_3)_2-$ | Öl |
| 44 | Cl | H | 6-Cl | 1 | $(CH_3)_3C-CH_2-$ | Öl |
| 45 | Cl | H | 6-Cl | 1 | $(CH_3)_2C=CH-$ | Öl |
| 46 | Cl | H | 6-Cl | 1 | 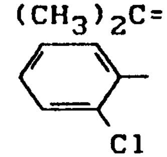 | 85 |

Tabelle 2 (Fortsetzung)

| Bsp. Nr. | X | Y | $Z_n$ | m | $R^1$ | Fp. °C |
|---|---|---|---|---|---|---|
| 47 | Cl | H | 6-Cl | 1 | 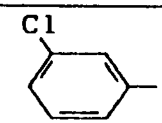 | 98 |
| 48 | Cl | H | 6-Cl | 1 | 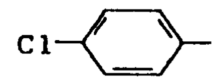 | 143 |
| 49 | $CH_3$ | $CH_3$ | 5-$CH_3$ | 1 | $CH_3$ | Öl |
| 50 | $CH_3$ | $CH_3$ | 5-$CH_3$ | 1 | $(CH_3)_2CH$- | |
| 51 | $CH_3$ | $CH_3$ | 5-$CH_3$ | 1 | $(CH_3)_3C$- | Öl |
| 52 | $CH_3$ | $CH_3$ | 5-$CH_3$ | 1 | $(CH_3)_2CHC(CH_3)_2$- | |
| 53 | $CH_3$ | $CH_3$ | 6-$CH_3$ | 1 | $CH_3$ | Öl |
| 54 | $CH_3$ | $CH_3$ | 6-$CH_3$ | 1 | $(CH_3)_2CH$- | Öl |
| 55 | $CH_3$ | $CH_3$ | 6-$CH_3$ | 1 | $(CH_3)_3C$- | Öl |
| 56 | $CH_3$ | $CH_3$ | 6-$CH_3$ | 1 | $(CH_3)_2CH-(CH_3)_2$-C- | Öl |
| 57 | $CH_3$ | $CH_3$ | 6-$CH_3$ | 1 | $C_4H_9$-CH- <br> \| <br> $C_2H_5$ | Öl |

Tabelle 2 (Fortsetzung)

| Bsp. Nr. | X | Y | $Z_n$ | m | $R^1$ | Fp. °C |
|---|---|---|---|---|---|---|
| 58 | $CH_3$ | $CH_3$ | 6-$CH_3$ | 1 | $(CH_3)_3C-CH_2-$ | Öl |
| 59 | $CH_3$ | $CH_3$ | 6-$CH_3$ | 1 | $CH_2=CH(CH_2)_7-$ | Öl |
| 60 | $CH_3$ | $CH_3$ | 6-$CH_3$ | 1 | $(CH_3)_2-CH-CH_2-$ | Öl |
| 61 | $CH_3$ | $CH_3$ | 6-$CH_3$ | 1 | 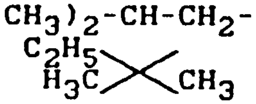 | Öl |
| 62 | $CH_3$ | $CH_3$ | 6-$CH_3$ | 1 | 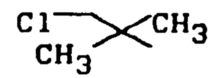 | Öl |
| 63 | $CH_3$ | $CH_3$ | 6-$CH_3$ | 1 | 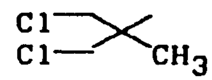 | 105 |
| 64 | $CH_3$ | $CH_3$ | 6-$CH_3$ | 1 | 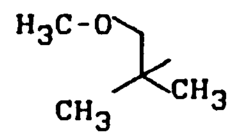 | Öl |
| 65 | $CH_3$ | $CH_3$ | 6-$CH_3$ | 1 | 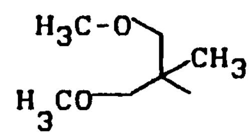 | Öl |
| 66 | $CH_3$ | $CH_3$ | 6-$CH_3$ | 1 | 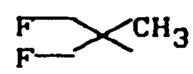 | Öl |

Tabelle 2 (Fortsetzung)

| Bsp. Nr. | X | Y | $Z_n$ | m | $R^1$ | Fp. °C |
|---|---|---|---|---|---|---|
| 67 | $CH_3$ | $CH_3$ | 6-$CH_3$ | 1 | 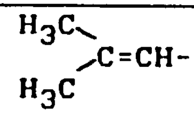 | Öl |
| 68 | $CH_3$ | $CH_3$ | 6-$CH_3$ | 1 | $H_3C-S-CH_2-$ | 105 |
| 69 | $CH_3$ | $CH_3$ | 6-$CH_3$ | 1 | 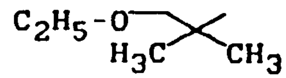 | Öl |
| 70 | $CH_3$ | $CH_3$ | 6-$CH_3$ | 1 | 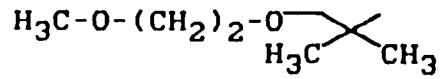 | Öl |
| 71 | $CH_3$ | $CH_3$ | 6-$CH_3$ | 1 | 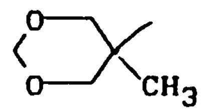 | 120 |
| 72 | $CH_3$ | $CH_3$ | 6-$CH_3$ | 1 | 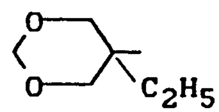 | Öl |
| 73 | $CH_3$ | $CH_3$ | 6-$CH_3$ | 1 | 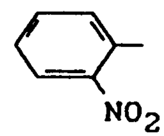 | 115 |

Tabelle 2 (Fortsetzung)

| Bsp. Nr. | X | Y | $Z_n$ | m | $R^1$ | Fp. °C |
|---|---|---|---|---|---|---|
| 74 | $CH_3$ | $CH_3$ | 6-$CH_3$ | 1 | 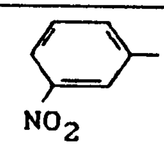 | 106 |
| 75 | $CH_3$ | $CH_3$ | 6-$CH_3$ | 1 | 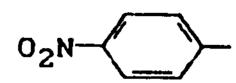 | 120 |
| 76 | $CH_3$ | $CH_3$ | 6-$CH_3$ | 1 | 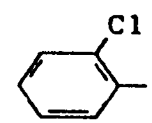 | Öl |
| 77 | $CH_3$ | $CH_3$ | 6-$CH_3$ | 1 | 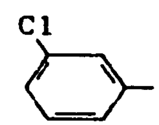 | Öl |
| 78 | $CH_3$ | $CH_3$ | 6-$CH_3$ | 1 | 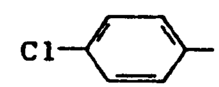 | 73 |

Tabelle 2 (Fortsetzung)

| Bsp. Nr. | X | Y | $Z_n$ | m | $R^1$ | Fp. °C |
|---|---|---|---|---|---|---|
| 79 | $CH_3$ | $CH_3$ | 6-$CH_3$ | 1 | 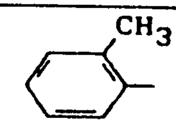 | 118 |
| 80 | $CH_3$ | $CH_3$ | 6-$CH_3$ | 1 | 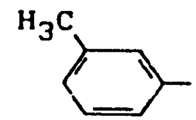 | 108 |
| 81 | $CH_3$ | $CH_3$ | 6-$CH_3$ | 1 | 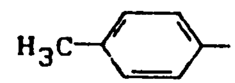 | Öl |
| 82 | $CH_3$ | $CH_3$ | 6-$CH_3$ | 1 | 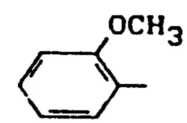 | 122 |
| 83 | $CH_3$ | $CH_3$ | 6-$CH_3$ | 1 | 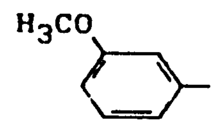 | 102 |

Tabelle 2 (Fortsetzung)

| Bsp. Nr. | X | Y | $Z_n$ | m | $R^1$ | Fp. °C |
|---|---|---|---|---|---|---|
| 84 | $CH_3$ | $CH_3$ | $6-CH_3$ | 1 | 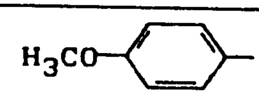 | Öl |
| 85 | Cl | Cl | - | 2 | $CH_3-$ | 120 |
| 86 | Cl | Cl | - | 2 | $(CH_3)_2CH-$ | 68 |
| 87 | Cl | Cl | - | 2 | $(CH_3)_3C-$ | 94 |
| 88 | Cl | Cl | - | 2 | $(CH_3)_2CH-C(CH_3)_2-$ | 62 |
| 89 | Cl | Cl | - | 2 | 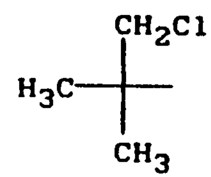 | Öl |
| 90 | Cl | Cl | - | 2 | 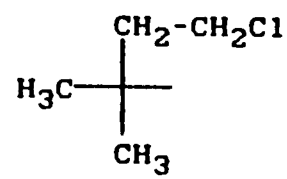 | 125 |

Tabelle 2 (Fortsetzung)

| Bsp. Nr. | X | Y | $Z_n$ | m | $R^1$ | Fp. °C |
|---|---|---|---|---|---|---|
| 91 | Cl | H | 6-Cl | 2 | $CH_3$- | 120 |
| 92 | Cl | H | 6-Cl | 2 | $(CH_3)_2CH$- | 82 |
| 93 | Cl | H | 6-Cl | 2 | $(CH_3)_3C$- | 110 |
| 94 | Cl | H | 6-Cl | 2 | $(CH_3)_2CH-C(CH_3)_2$- | 92 |
| 95 | Cl | H | 6-Cl | 2 | $CH_3-S-CH_2$- | 126 |
| 96 | Cl | H | 6-Cl | 2 | 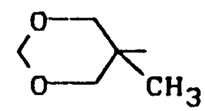 | 150 |
| 97 | Cl | H | 6-Cl | 2 | 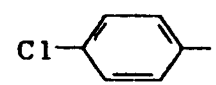 | 150 |
| 98 | Cl | H | 6-Cl | 2 | 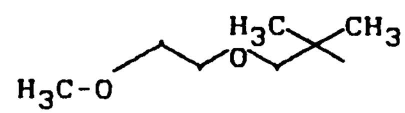 | 106 |
| 99 | Cl | H | 6-Cl | 2 | 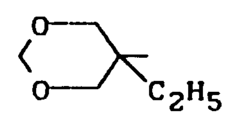 | 162 |

**Tabelle 2** (Fortsetzung)

| Bsp. Nr. | X | Y | $Z_n$ | m | $R^1$ | Fp. °C |
|---|---|---|---|---|---|---|
| 100 | Cl | H | 6-Cl | 2 | 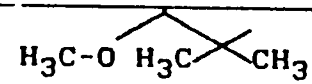 | 130 |
| 101 | Cl | H | 6-Cl | 2 | 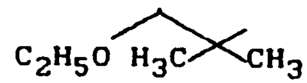 | 107 |
| 102 | Cl | H | 6-Cl | 2 | 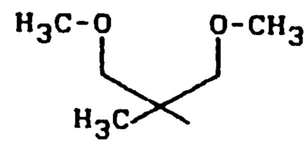 | 107 |
| 103 | Cl | H | 6-Cl | 2 | $(H_3C-O-CH_2)_3C-$ | 105 |
| 104 | Cl | H | 6-Cl | 2 | 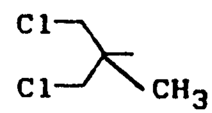 | 126 |
| 105 | Cl | H | 6-Cl | 2 | 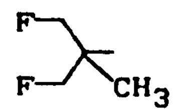 | 114 |

**Tabelle 2** (Fortsetzung)

| Bsp. Nr. | X | Y | $Z_n$ | m | $R^1$ | Fp. °C |
|---|---|---|---|---|---|---|
| 106 | Cl | H | 6-Cl | 2 | 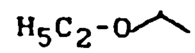 | Öl |
| 107 | Cl | H | 6-Cl | 2 | 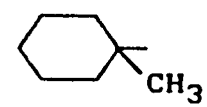 | 136 |
| 108 | Cl | H | 6-Cl | 2 | 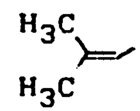 | Öl |
| 109 | Cl | H | 6-Cl | 2 | $(CH_3)_3C-CH_2-$ | Öl |
| 110 | Cl | H | 6-Cl | 2 | 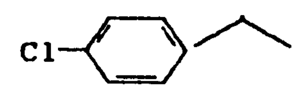 | 122 |
| 111 | Cl | H | 6-Cl | 2 | $CH_2=CH(CH_2)_7-$ | Öl |
| 112 | Cl | H | 6-Cl | 2 | 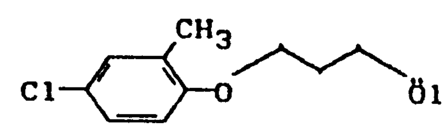 | Öl |

<u>Tabelle 2</u> (Fortsetzung)

| Bsp. Nr. | X | Y | $Z_n$ | m | $R^1$ | Fp. °C |
|---|---|---|---|---|---|---|
| 113 | Cl | H | 6-Cl | 2 | 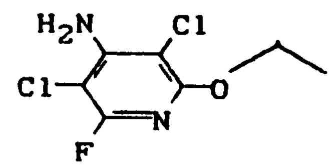 | |
| 114 | Cl | H | 6-F | 2 | $CH_3$- | Öl |
| 115 | Cl | H | 6-F | 2 | $(CH_3)_3C$- | 102 |
| 116 | $CH_3$ | $CH_3$ | - | 2 | $CH_3$- | Öl |
| 117 | $CH_3$ | $CH_3$ | - | 2 | $(CH_3)_2CH$- | Öl |
| 118 | $CH_3$ | $CH_3$ | - | 2 | $(CH_3)_3C$- | 65 |
| 119 | $CH_3$ | $CH_3$ | - | 2 | $(CH_3)_2CH-(CH_3)_2-C$- | Öl |
| 120 | $CH_3$ | t-$C_4H_9$ | - | 2 | $CH_3$- | Öl |
| 121 | $CH_3$ | t-$C_4H_9$ | - | 2 | $(CH_3)_2CH$- | Öl |
| 122 | $CH_3$ | t-$C_4H_9$ | - | 2 | $(CH_3)_3C$- | Öl |

Tabelle 2 (Fortsetzung)

| Bsp. Nr. | X | Y | $Z_n$ | m | $R^1$ | Fp. °C |
|---|---|---|---|---|---|---|
| 123 | $CH_3$ | $CH_3$ | 5-$CH_3$ | 2 | $CH_3$ | 102 |
| 124 | $CH_3$ | $CH_3$ | 5-$CH_3$ | 2 | $(CH_3)_2CH-$ | 88 |
| 125 | $CH_3$ | $CH_3$ | 5-$CH_3$ | 2 | $(CH_3)_3C-$ | 103 |
| 126 | $CH_3$ | $CH_3$ | 5-$CH_3$ | 2 | $(CH_3)_2CH-(CH_3)_2C-$ | Öl |
| 127 | $CH_3$ | $CH_3$ | 5-$CH_3$ | 2 | $C_4H_9CH-$<br>\|<br>$C_2H_5$ | |
| 128 | $CH_3$ | $CH_3$ | 6-$CH_3$ | 2 | $CH_3-$ | Öl |
| 129 | $CH_3$ | $CH_3$ | 6-$CH_3$ | 2 | $(CH_3)_2CH-$ | Öl |
| 130 | $CH_3$ | $CH_3$ | 6-$CH_3$ | 2 | $(CH_3)_3C-$ | 93 |
| 131 | $CH_3$ | $CH_3$ | 6-$CH_3$ | 2 | $(CH_3)_2CH-(CH_3)_2C-$ | 68 |

Tabelle 2 (Fortsetzung)

| Bsp. Nr. | X | Y | $Z_n$ | m | $R^1$ | Fp. °C |
|---|---|---|---|---|---|---|
| 132 | $CH_3$ | $CH_3$ | 6-$CH_3$ | 2 | 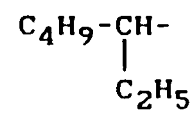 | Öl |
| 133 | $CH_3$ | $CH_3$ | 6-$CH_3$ | 2 | $CH_3S-CH_2-$ | 93 |
| 134 | $CH_3$ | $CH_3$ | 6-$CH_3$ | 2 | $C_2H_5-O-CH_2-$ | Öl |
| 135 | $CH_3$ | $CH_3$ | 6-$CH_3$ | 2 | 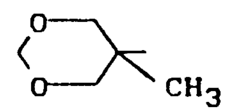 | 100 |
| 137 | $CH_3$ | $CH_3$ | 6-$CH_3$ | 2 | 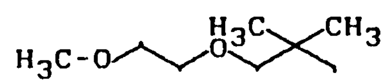 | Öl |
| 138 | $CH_3$ | $CH_3$ | 6-$CH_3$ | 2 | 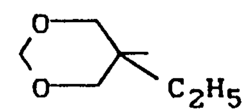 | 100 |
| 139 | $CH_3$ | $CH_3$ | 6-$CH_3$ | 2 | 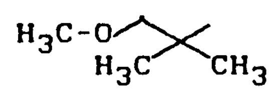 | 52 |

Tabelle 2 (Fortsetzung)

| Bsp. Nr. | X | Y | $Z_n$ | m | $R^1$ | Fp. °C |
|---|---|---|---|---|---|---|
| 139 | $CH_3$ | $CH_3$ | 6-$CH_3$ | 2 | 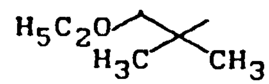 | 71 |
| 140 | $CH_3$ | $CH_3$ | 6-$CH_3$ | 2 | 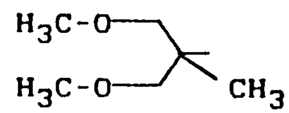 | Öl |
| 141 | $CH_3$ | $CH_3$ | 6-$CH_3$ | 2 | $(H_3C-O-CH_2)_3C-$ | 108 |
| 142 | $CH_3$ | $CH_3$ | 6-$CH_3$ | 2 | 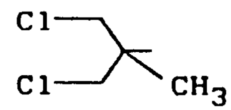 | 112 |
| 143 | $CH_3$ | $CH_3$ | 6-$CH_3$ | 2 | 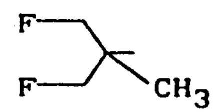 | 83 |
| 144 | $CH_3$ | $CH_3$ | 6-$CH_3$ | 2 | 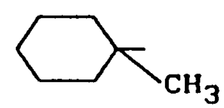 | 103 |

Tabelle 2 (Fortsetzung)

| Bsp. Nr. | X | Y | $Z_n$ | m | $R^1$ | Fp. °C |
|---|---|---|---|---|---|---|
| 145 | $CH_3$ | $CH_3$ | 6-$CH_3$ | 2 | 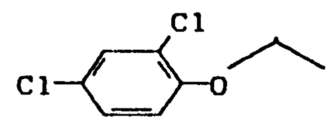 | 110 |
| 146 | $CH_3$ | $CH_3$ | 6-$CH_3$ | 2 | $CH_2$=CH-$(CH_2)_8$- | Öl |
| 147 | $CH_3$ | $CH_3$ | 6-$CH_3$ | 2 | 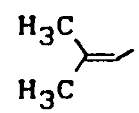 | Öl |
| 148 | $CH_3$ | $CH_3$ | 6-$CH_3$ | 2 | $(CH_3)_3C$-$CH_2$- | |
| 149 | $CH_3$ | $CH_3$ | 6-$CH_3$ | 2 | 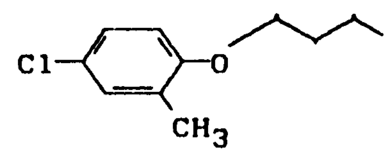 | Öl |
| 150 | $CH_3$ | $CH_3$ | 6-$CH_3$ | 2 | 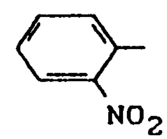 | 118 |

Tabelle 2 (Fortsetzung)

| Bsp. Nr. | X | Y | $Z_n$ | m | $R^1$ | Fp. °C |
|---|---|---|---|---|---|---|
| 151 | $CH_3$ | $CH_3$ | 6-$CH_3$ | 2 | 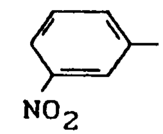 | 147 |
| 152 | $CH_3$ | $CH_3$ | 6-$CH_3$ | 2 | 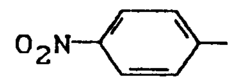 | 88 |
| 153 | $CH_3$ | $CH_3$ | 6-$CH_3$ | 2 | 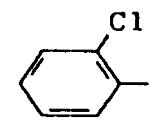 | 75 |
| 154 | $CH_3$ | $CH_3$ | 6-$CH_3$ | 2 | 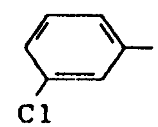 | 98 |
| 155 | $CH_3$ | $CH_3$ | 6-$CH_3$ | 2 | 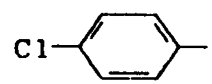 | 117 |

Tabelle 2 (Fortsetzung)

| Bsp. Nr. | X | Y | $Z_n$ | m | $R^1$ | Fp. °C |
|---|---|---|---|---|---|---|
| 156 | $CH_3$ | $CH_3$ | 6-$CH_3$ | 2 | 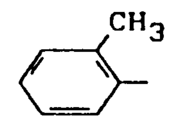 | 84 |
| 157 | $CH_3$ | $CH_3$ | 6-$CH_3$ | 2 | 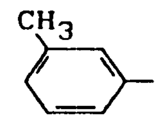 | 96 |
| 158 | $CH_3$ | $CH_3$ | 6-$CH_3$ | 2 | 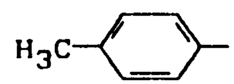 | 125 |
| 159 | $CH_3$ | $CH_3$ | 6-$CH_3$ | 2 | 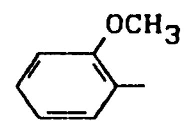 | 147 |
| 160 | $CH_3$ | $CH_3$ | 6-$CH_3$ | 2 | 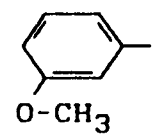 | 98 |

<u>Tabelle 2</u> (Fortsetzung)

| Bsp. Nr. | X | Y | $Z_n$ | m | $R^1$ | Fp. °C |
|---|---|---|---|---|---|---|
| 161 | $CH_3$ | $CH_3$ | 6-$CH_3$ | 2 | 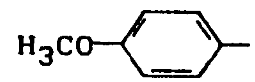 | 102 |
| 162 | $CH_3$ | $CH_3$ | 6-$CH_3$ | 2 | 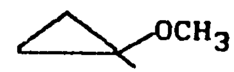 | 83 |
| 163 | $CH_3$ | $CH_3$ | 6-$CH_3$ | 2 | 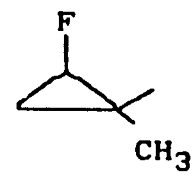 | Öl |
| 164 | $CH_3$ | $CH_3$ | 6-$CH_3$ | 2 | 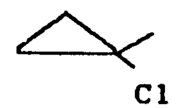 | 103 |
| 165 | $CH_3$ | $CH_3$ | 6-$CH_3$ | 2 | 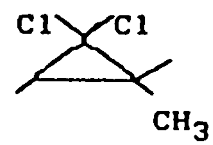 | Öl |

**Tabelle 2** (Fortsetzung)

| Bsp. Nr. | X | Y | $Z_n$ | m | $R^1$ | Fp. °C |
|---|---|---|---|---|---|---|
| 166 | $CH_3$ | $CH_3$ | 6-$CH_3$ | 2 | 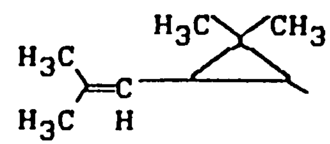 | Öl |
| 167 | Cl | Cl | 6-Cl | 2 | $CH_3$- | 123 |
| 168 | Cl | Cl | 6-Cl | 2 | $(CH_3)_3C$- | 172 |
| 169 | Cl | $CF_3$ | 6-F | 2 | $(CH_3)_3C$- | 122 |
| 170 | Cl | $CF_3$ | 6-Cl | 2 | $CH_3$- | 133 |
| 171 | Cl | $CF_3$ | 6-Cl | 2 | $(CH_3)_3C$- | 128 |
| 172 | i-$C_3H_7$ | i-$C_3H_7$ | 6-i-$C_3H_7$ | 2 | $CH_3$- | 125 |
| 173 | i-$C_3H_7$ | i-$C_3H_7$ | 6-i-$C_3H_7$ | 2 | $(CH_3)_3C$- | 178 |
| 174 | $CH_3$ | F | 6-$CH_3$ | 2 | $CH_3$- | 85 |
| 175 | $CH_3$ | F | 6-$CH_3$ | 2 | $(CH_3)_3C$- | 110 |
| 176 | $CH_3$ | $CH_3$ | 6-F | 2 | $CH_3$- | Öl |
| 177 | $CH_3$ | $CH_3$ | 6-F | 2 | $(CH_3)_3C$- | Öl |
| 178 | $CH_3$ | t-$C_4H_9$ | 6-$CH_3$ | 2 | $CH_3$- | 109 |

Tabelle 2 (Fortsetzung)

| Bsp. Nr. | X | Y | $Z_n$ | m | $R^1$ | Fp. °C |
|---|---|---|---|---|---|---|
| 179 | $CH_3$ | $t-C_4H_9$ | $6-CH_3$ | 2 | $(CH_3)_2CH-$ | 92 |
| 180 | $CH_3$ | $t-C_4H_9$ | $6-CH_3$ | 2 | $(CH_3)_3C-$ | 161 |
| 181 | $CH_3$ | $t-C_4H_9$ | $6-CH_3$ | 2 | $(CH_3)_2CH-C(CH_3)_2-$ | 99 |
| 182 | Cl | H | 3.6-di-Cl | 2 | $CH_3-$ | 127 |
| 183 | Cl | H | 3.6-di-Cl | 2 | $(CH_3)_3C-$ | Öl |
| 184 | $CH_3$ | $CH_3$ | $3.5-di-CH_3$ | 2 | $CH_3-$ | 120 |
| 185 | $CH_3$ | $CH_3$ | $3.5-di-CH_3$ | 2 | $(CH_3)_3C-$ | 107 |
| 186 | $CH_3$ | $CH_3$ | $3.6-di-CH_3$ | 2 | $CH_3-$ | Öl |
| 187 | $CH_3$ | $CH_3$ | $3.6-di-CH_3$ | 2 | $(CH_3)_3C-$ | 97 |
| 188 | $CH_3$ | H | $3.5.6-tri-CH_3$ | 2 | $CH_3-$ | Öl |
| 189 | $CH_3$ | H | $3.5.6-tri-CH_3$ | 2 | $(CH_3)_3C-$ | 82 |
| 190 | $CH_3$ | $CH_3$ | $3-Br,6-CH_3$ | 2 | $CH_3-$ | Öl |
| 191 | $CH_3$ | $CH_3$ | $3-Br,6-CH_3$ | 2 | $(CH_3)_2CH-$ | Öl |
| 192 | $CH_3$ | $CH_3$ | $3-Br,6-CH_3$ | 2 | $(CH_3)_3C-$ | Öl |
| 193 | $CH_3$ | $CH_3$ | $3-Br,6-CH_3$ | 2 | $(CH_3)_2CH-C(CH_3)_2-$ | Öl |

**Tabelle 2 (Fortsetzung)**

| Bsp. Nr. | X | Y | $Z_n$ | m | $R^1$ | Fp. °C |
|---|---|---|---|---|---|---|
| 194 | $CH_3$ | $CH_3$ | tri-$CH_3$ | 2 | $CH_3$- | 136 |
| 195 | $CH_3$ | $CH_3$ | tri-$CH_3$ | 2 | $(CH_3)_2CH$- | 79 |
| 196 | $CH_3$ | $CH_3$ | tri-$CH_3$ | 2 | $(CH_3)_3C$- | 98 |

**Tabelle 3**

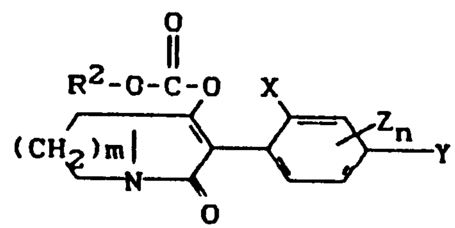

(Ic)

| Bsp. Nr. | X | Y | $Z_n$ | m | $R^2$ | Fp. °C |
|---|---|---|---|---|---|---|
| 197 | Cl | Cl | - | 1 | $(CH_3)_2CH-$ | |
| 198 | Cl | Cl | - | 1 | 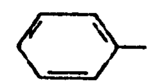 | |
| 199 | Cl | H | 6-Cl | 1 | $CH_3-$ | |
| 200 | Cl | H | 6-Cl | 1 | $(CH_3)_2CH-$ | 115 |
| 201 | Cl | H | 6-Cl | 1 | $(CH_3)_3-C-CH_2-$ | 92 |
| 202 | Cl | H | 6-Cl | 1 | $H_5C_2O$ 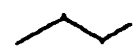 | |
| 203 | Cl | H | 6-Cl | 1 | $(CH_3)_2CH-CH_2-$ | Öl |
| 204 | Cl | H | 6-Cl | 1 | $H_3C-(CH_2)_2-CH(CH_3)-$ | Öl |

**Tabelle 3** (Fortsetzung)

| Bsp. Nr. | X | Y | $Z_n$ | m | $R^2$ | Fp. °C |
|---|---|---|---|---|---|---|
| 205 | Cl | H | 6-Cl | 1 | $(CH_3)_2CH-CH-$<br>\|<br>$CH_3$ | 80 |
| 206 | Cl | H | 6-Cl | 1 | $H_5C_2-CH-$<br>\|<br>$CH_3$ | 46 |
| 207 | Cl | H | 6-Cl | 1 | 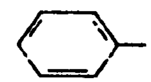 | |
| 208 | $CH_3$ | $CH_3$ | 5-$CH_3$ | 1 | $(CH_3)_2CH-$ | |
| 209 | $CH_3$ | $CH_3$ | 5-$CH_3$ | 1 | $(CH_3)_2CH-CH_2-$ | |
| 210 | $CH_3$ | $CH_3$ | 5-$CH_3$ | 1 | $H_5C_2-CH-$<br>\|<br>$CH_3$ | |
| 211 | $CH_3$ | $CH_3$ | 5-$CH_3$ | 1 | $H_5C_2-O$ 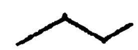 | |
| 212 | $CH_3$ | $CH_3$ | 5-$CH_3$ | 1 | 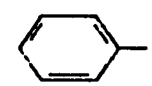 | |

**Tabelle 3** (Fortsetzung)

| Bsp. Nr. | X | Y | $Z_n$ | m | $R^2$ | Fp. °C |
|---|---|---|---|---|---|---|
| 213 | $CH_3$ | $CH_3$ | $6-CH_3$ | 1 | $CH_3-$ | Öl |
| 214 | $CH_3$ | $CH_3$ | $6-CH_3$ | 1 | $C_2H_5-$ | Öl |
| 215 | $CH_3$ | $CH_3$ | $6-CH_3$ | 1 | $(CH_3)CH-$ | 54 |
| 216 | $CH_3$ | $CH_3$ | $6-CH_3$ | 1 | $(CH_3)_2CH-CH_2-$ | Öl |
| 217 | $CH_3$ | $CH_3$ | $6-CH_3$ | 1 | 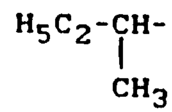 | 95 |
| 218 | $CH_3$ | $CH_3$ | $6-CH_3$ | 1 | 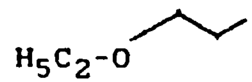 | Öl |
| 219 | $CH_3$ | $CH_3$ | $6-CH_3$ | 1 | 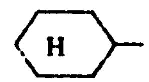 | Öl |
| 220 | $CH_3$ | $CH_3$ | $6-CH_3$ | 1 | $(CH_3)_3C-$ | Öl |
| 221 | $CH_3$ | $CH_3$ | $6-CH_3$ | 1 | 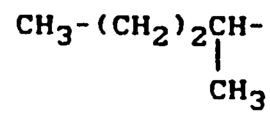 | 98 |

**Tabelle 3** (Fortsetzung)

| Bsp. Nr. | X | Y | $Z_n$ | m | $R^2$ | Fp. °C |
|---|---|---|---|---|---|---|
| 222 | $CH_3$ | $CH_3$ | 6-$CH_3$ | 1 | $(CH_3)_3C-CH_2-$ | Öl |
| 223 | $CH_3$ | $CH_3$ | 6-$CH_3$ | 1 | $(CH_3)_2-CH-CH<^{CH_3}$ | Öl |
| 224 | Cl | Cl | - | 2 | $(CH_3)_2CH-$ | |
| 225 | Cl | Cl | - | 2 | $(CH_3)_2CH-CH_2-$ | |
| 226 | Cl | Cl | - | 2 | 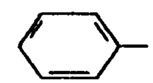 | |
| 227 | Cl | Cl | - | 2 | $C_2H_5-O-C_2H_4-O-C_2H_4-$ | |
| 228 | Cl | H | 6-Cl | 2 | $H_3C-$ | |
| 229 | Cl | H | 6-Cl | 2 | $(CH_3)_2CH-$ | 121 |
| 230 | Cl | H | 6-Cl | 2 | $(CH_3)_2CHCH_2-$ | 108 |
| 231 | Cl | H | 6-Cl | 2 | $H_5C_2-CH-$<br>$CH_3$ | 100 |

**Tabelle 3** (Fortsetzung)

| Bsp. Nr. | X | Y | $Z_n$ | m | $R^2$ | Fp. °C |
|---|---|---|---|---|---|---|
| 232 | Cl | H | 6-Cl | 2 | $H_5C_2$-O 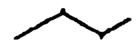 | Öl |
| 233 | Cl | H | 6-Cl | 2 | $H_5C_2$-O 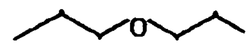 | Öl |
| 234 | Cl | H | 6-Cl | 2 | 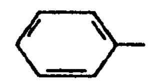 | 138 |
| 235 | $CH_3$ | $CH_3$ | 5-$CH_3$ | 2 | $H_3C$- | |
| 236 | $CH_3$ | $CH_3$ | 5-$CH_3$ | 2 | $(CH_3)_2CH$- | |
| 237 | $CH_3$ | $CH_3$ | 5-$CH_3$ | 2 | $(CH_3)_2CH-CH_2$- | |
| 238 | $CH_3$ | $CH_3$ | 5-$CH_3$ | 2 | $H_5C_2$-CH-<br>\|<br>$CH_3$ | |
| 239 | $CH_3$ | $CH_3$ | 5-$CH_3$ | 2 | $H_5C_2$-O 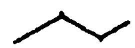 | |
| 240 | $CH_3$ | $CH_3$ | 5-$CH_3$ | 2 | 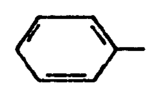 | |

**Tabelle 3 (Fortsetzung)**

| Bsp. Nr. | X | Y | $Z_n$ | m | $R^2$ | Fp. °C |
|---|---|---|---|---|---|---|
| 241 | $CH_3$ | $CH_3$ | 6-$CH_3$ | 2 | $H_3C$- | 105 |
| 242 | $CH_3$ | $CH_3$ | 6-$CH_3$ | 2 | $C_2H_5$- | 102 |
| 243 | $CH_3$ | $CH_3$ | 6-$CH_3$ | 2 | $(CH_3)_2CH$- | Öl |
| 244 | $CH_3$ | $CH_3$ | 6-$CH_3$ | 2 | $(CH_3)_2CH$-$CH_2$- | Öl |
| 245 | $CH_3$ | $CH_3$ | 6-$CH_3$ | 2 | $H_5C_2$-CH-<br>$CH_3$ | Öl |
| 246 | $CH_3$ | $CH_3$ | 6-$CH_3$ | 2 | $H_5C_2$-O 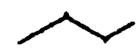 | Öl |
| 247 | $CH_3$ | $CH_3$ | 6-$CH_3$ | 2 | $H_5C_2$-O 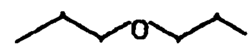 | Öl |
| 248 | $CH_3$ | $CH_3$ | 6-$CH_3$ | 2 | 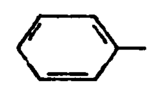 | 110 |
| 249 | $CH_3$ | $CH_3$ | 6-$CH_3$ | 2 | $(CH_3)_3C$- | 109 |
| 250 | $CH_3$ | $CH_3$ | 6-$CH_3$ | 2 | $CH_3$-$(CH_2)_2$-CH<br>$CH_3$ | Öl |

<u>Tabelle 3</u> (Fortsetzung)

| Bsp. Nr. | X | Y | $Z_n$ | m | $R^2$ | Fp. °C |
|---|---|---|---|---|---|---|
| 251 | $CH_3$ | $CH_3$ | 6-$CH_3$ | 2 | $(CH_3)_2CH-CH-$<br>\|<br>$CH_3$ | Öl |
| 252 | $CH_3$ | $CH_3$ | 6-$CH_3$ | 2 | $(CH_3)_3C-CH_2-$ | Öl |
| 253 | $CH_3$ | $CH_3$ | 6-$CH_3$ | 2 | 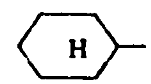 | 142 |
| 254 | $CH_3$ | t-$C_4H_9$ | - | 2 | $C_2H_5-CH-$<br>\|<br>$CH_3$ | Öl |
| 255 | $CH_3$ | F | 6-$CH_3$ | 2 | $(CH_3)_2CH-$ | Öl |

Beispiel 256

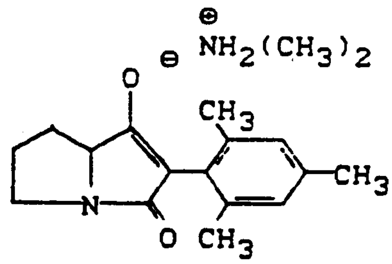

2,57 g (10 mMol) 3 – (2,4,6 – Trimethyl – phenyl) – 1,5 – trimethylen – pyrrolidin – 2,4 – dion werden in 50 ml absoluten THF (Tetrahydrofuran) suspendiert und anschließend Dimethylamin durchgeleitet, bis die Gasaufnahme beendet ist. Nach dem Rotationsverdampfen des THF wird im Vakuum bei 70 ° C getrocknet.
Ausbeute: 2,69 g (89,7 % der Theorie) Fp. 62 ° C

In entsprechender Weise wurden Verbindungen der Formel (Id) hergestellt:

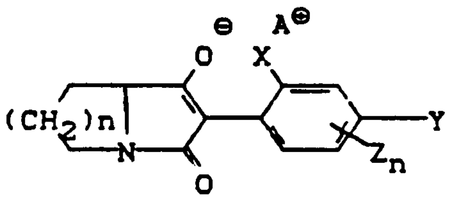

(Id)

Tabelle 4

| Bsp. Nr. | X | Y | $Z_n$ | m | $A^{\oplus}$ | Fp. ° C |
|---|---|---|---|---|---|---|
| 257 | $CH_3$ | $CH_3$ | $CH_3$ | 1 | $Na^{\oplus}$ | <230 |
| 258 | $CH_3$ | $CH_3$ | $CH_3$ | 1 | $NH_3^{\oplus}$ | 228 |

Zwischenprodukte

Beispiel 5'

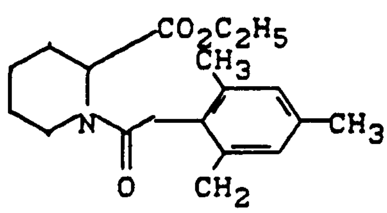

Zu 182 ml (1,157 Mol) Pipecolinsäureethylester und 162 ml (1,157 Mol) Triethylamin in 1.200 ml absolutem Tetrahydrofuran (THF) tropft man bei 0 bis 10 ° C 223 g (1,125 Mol) Mesitylenessigsäurechlorid und rührt 1 Stunde bei Raumtemperatur nach. Nach Einrühren in 5 l Eiswasser und 500 ml 1 N HCl wird das Produkt abgesaugt, mit Wasser nachgewaschen und bei 50 ° C im Vakuum über $P_2O_5$ getrocknet. Man erhält 342,3 g (95,2 % der Theorie) N – (2,4,6 – Trimethylphenylacetyl) – piperidin – 2 – carbonsäureethylester.

Beispiel 37'

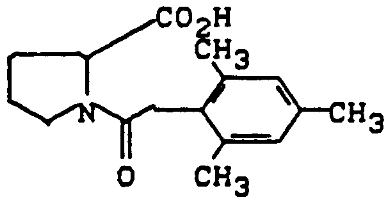

Zu 115 g (1 Mol) L-Prolin in 1 l Wasser gibt man 20 g (0,5 Mol) NaOH-Plätzchen. Bei einer Temperatur unter 40 °C werden synchron 60 g (1,5 Mol) NaOH in 300 ml Wasser und 197,6 g (1 Mol) Mesitylenessigsäurechlorid zugetropft und 1 Stunde nachgerührt. Anschließend wird bei 5 bis 20 °C mit konzentrierter Salzsäure angesäuert, das Produkt abgesaugt und im Vakuum bei 70 °C über $P_2O_5$ getrocknet.
Ausbeute: 262 g (95,3 % der Theorie) N-2,4,6-Trimethylphenylacetylprolin Fp. 156 °C

Beispiel 26'

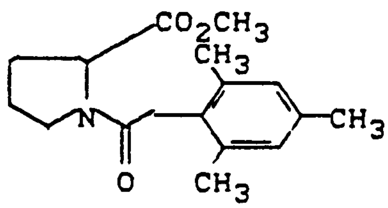

137,5 g (0,5 Mol) N-2,4,6-Trimethylphenylacetyl-prolin werden in 500 ml Methanol gelöst. Nach Zugabe von 73 ml (0,55 Mol) Dimethoxypropan und 4,75 g (25 mMol) p-Toluolsulfonsäure-monohydrat erhitzt man 2 Stunden unter Rückfluß. Nach Einrotieren wird der Rückstand in Methylenchlorid aufgenommen, mit Natriumhydrogencarbonat-Lösung gewaschen, die Methylenchlorid-Phase getrocknet und einrotiert. Nach Umkristallisieren aus Methylenchlorid/Methyl-tert.-butylether/n-Hexan wurden 107,9 g (74,7 %) N-(2,4,6-Trimethylphenylacetyl)-pyrrolidin-2-carbonsäuremethylester erhalten. Fp. 74 °C.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex

lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodorus spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Charakteristisch für die erfindungsgemäßen Verbindungen ist, daß sie eine selektive Wirksamkeit gegen monokotyle Unkräuter im Vor- und Nachlaufverfahren (Pre- und Postemergence) bei guter Kulturflanzenverträglichkeit aufweisen.

Die erfindungsgemäßen Wirkstoffe können z.B. bei den folgenden Pflanzen verwendet werden:
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfen-

anlagen und zur selektiven Unkrautbekämpfung in einjährigen Kulturen eingesetzt werden.

Dabei zeigen die erfindungsgemäßen Wirkstoffe neben einer hervorragenden Wirkung gegen Schadpflanzen gute Verträglichkeit gegenüber wichtigen Kulturpflanzen, wie z. B. Weizen, Baumwolle, Sojabohnen, Citrusfrüchten und Zuckerrüben, und können daher als selektive Unkrautbekämpfungsmittel eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden. Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäß verwendbaren Wirkstoffe eignen sich auch zur Bekämpfung von Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw.

erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die erfindungsgemäßen Verbindungen der Formel (I) weisen antimikrobielle, insbesondere starke antibakterielle und antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullenvorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelantine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen, z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat,Ethylacetat, Benzylalkohol, Benzylbenzoat, Propylenglykol, 1,3-Buty lenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccarin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichenund zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel A

Tetranychus-Test (resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik (88).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Bei diesem Test zeigen zeigen z.B. die folgenden Verbindungen der Herstellungsbeispiele überlegene Wirksamkeit gegenüber dem Stand der Technik: (1), (13).

Beispiel C

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Bei diesem Test zeigen z.B. die folgenden Verbindungen der Herstellungsbeispiele überlegene Wirksamkeit gegenüber dem Stand der Technik: (1), (13).

Beispiel D

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad

bestimmt.

Bei diesem Test zeigen z.B. die folgenden Verbindungen der Herstellungsbeispiele eine stark ausgeprägte Wirksamkeit: 7, 54, 58, 62, 64, 67, 68, 213, 215, 216, 217, 222.

Beispiel E

Test mit Psoroptes ovis

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die gewünschte Konzentration.

Etwa 10 – 25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bie diesem Test zeigen z.B. die folgenden Verbindungen der Herstellungsbeispiele eine stark ausgeprägte Wirksamkeit: 39, 55, 57, 62, 87, 89, 167, 201, 245.

Beispiel F

Antimykotische in-vitro-Wirksamkeit

Versuchsbeschreibung:

Die in-vitro-Prüfungen wurden mit Keiminokula von durchschnittlich 1 x $10^4$ Keimen/ml Substrat durchgeführt. Als Nährmedium diente Yeast Nitrogen Base-Medium für Hefen und Kimmig-Medium für Schimmelpilze und Dermatophyten.

Die Bebrütungstemperatur betrug 37 °C bei Hefen und 28 °C bei Schimmelpilzen und Dermatophyten, die Bebrütungsdauer lag bei 24 bis 96 Stunden bei Hefen und 96 bis 120 Stunden bei Dermatophyten und Schimmelpilzen.

Die Beurteilung der Fungizide erfolgt durch Ausplattieren und erneutes Bebrüten voll gehemmter Ansätze, wobei fungizide Konzentrationen weniger als 100 Keime c.f.n. (colony forming unit) pro ml enthalten.

In diesem Test zeigen die erfindungsgemäßen Verbindungen der Formel (I) gemäß den Herstellungsbeispielen 36, 40, 85, 91, 114, 116, 120, 167, 170, 174, 176, 178, 182, 184, 188, 194 eine stark ausgeprägte antimykotische Wirksamkeit.

## Patentansprüche

**1.** 3-Aryl-pyrrolidin-2,4-dion(e)-Derivate der Formel (I)

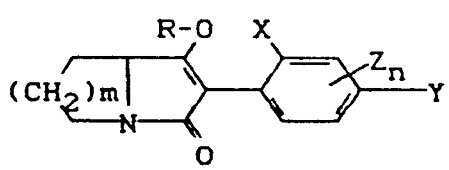

(I)

in welcher

X für $C_1-C_6$-Alkyl, Halogen, $C_1-C_6$-Alkoxy steht,
Y für Wasserstoff, $C_1-C_6$-Alkyl, Halogen, $C_1-C_6$-Alkoxy, $C_1-C_3$-Halogenalkyl steht,
Z für $C_1-C_6$-Alkyl, Halogen, $C_1-C_6$-Alkoxy steht,
m für eine Zahl von 1-4 steht,
n für eine Zahl von 0-3 steht,
R für Wasserstoff (Ia) oder für die Gruppen der Formel

$-CO-R^1$ (Ib) ,

$-CO-O-R^2$ (Ic)

oder

A (Id)

steht,
in welchen

A für ein Metallkationäquivalent oder für ein Ammoniumion steht,

$R^1$ für gegebenenfalls durch Halogen substituiertes: $C_1-C_{20}$-Alkyl, $C_2-C_{20}$-Alkenyl, $C_1-C_8$-Alkoxy-$C_1-C_8$-alkyl, $C_1-C_8$-Alkylthio-$C_1-C_8$-alkyl, $C_1-C_8$-Polyalkoxy-$C_2-C_8$-alkyl und Cycloalkyl mit 3-8 Ringatomen, das durch Sauerstoff und/oder Schwefel unterbrochen sein kann, steht,
für gegebenenfalls durch Halogen-, Nitro-, $C_1-C_6$-Alkyl-, $C_1-C_6$-Alkoxy-, $C_1-C_6$-Halogenalkyl-, $C_1-C_6$-Halogenalkoxy-substituiertes Phenyl;
für gegebenenfalls durch Halogen-, $C_1-C_6$-Alkyl, $C_1-C_6$-Alkoxy-, $C_1-C_6$-Halogenalkyl-, $C_1-C_6$-Halogenalkoxy-substituiertes Phenyl-$C_1-C_6$-alkyl steht,
für gegebenenfalls durch Halogen- und $C_1-C_6$-Alkyl-substituiertes Hetaryl steht,
für gegebenenfalls durch Halogen- und $C_1-C_6$-Alkyl-substituiertes Phenoxy-$C_1-C_6$-alkyl steht,
für gegebenenfalls durch Halogen, Amino und $C_1-C_6$-Alkyl-substituiertes Hetaryloxy-$C_1-C_6$-Alkyl steht,

$R^2$ für gegebenenfalls durch Halogen substituiertes: $C_1-C_{20}$-Alkyl, $C_2-C_{20}$-Alkenyl, $C_1-C_8$-Alkoxy-$C_2-C_8$-alkyl, $C_1-C_8$-Polyalkoxy-$C_2-C_8$-alkyl steht,
für gegebenenfalls durch Halogen-, $C_1-C_6$-Alkyl-, $C_1-C_6$-Alkoxy-, $C_1-C_6$-Halogenalkyl-substituiertes Phenyl steht,

sowie die enantiomeren Formen von Verbindungen der Formel (I).

**2.** 3-Aryl-pyrrolidin-2,4-dione der Formel (I) gemäß Anspruch 1,
in welcher

X für $C_1-C_4$-Alkyl, Halogen, $C_1-C_4$-Alkoxy steht,

Y für Wasserstoff, $C_1-C_6$-Alkyl, Halogen, $C_1-C_4$-Alkoxy, $C_1-C_2$-Halogenalkyl steht,

Z für $C_1-C_4$-Alkyl, Halogen, $C_1-C_4$-Alkoxy steht,

m für eine Zahl von 1-3 steht,

n für eine Zahl von 0-3 steht,

R für Wasserstoff (Ia) oder für die Gruppen der Formel

$-CO-R^1$ (Ib) ,
$-CO-O-R^2$ (Ic)

oder

A (Id)

steht,
in welchen

A für ein Metallkationäquivalent oder für ein Ammoniumion steht,

$R^1$ für gegebenenfalls durch Halogen substituiertes: $C_1-C_{16}$-Alkyl, $C_2-C_{16}$-Alkenyl, $C_1-C_6$-Alkoxy-$C_1-C_6$-alkyl, $C_1-C_6$-Alkylthio-$C_1-C_6$-alkyl, $C_1-C_6$-Polyalkoxy-$C_2-C_6$-alkyl und Cycloalkyl mit 3-7 Ringatomen, das durch 1-2 Sauerstoff- und/oder Schwefelatome unterbrochen sein kann steht,
für gegebenenfalls durch Halogen-, Nitro-, $C_1-C_4$-Alkyl-, $C_1-C_4$-Alkoxy-, $C_1-C_3$-Halogenalkyl-, $C_1-C_3$-Halogenalkoxy-substituiertes Phenyl steht,
für gegebenenfalls durch Halogen-, $C_1-C_4$-Alkyl-, $C_1-C_4$-Alkoxy-, $C_1-C_3$-Halogenalkyl-, $C_1-C_3$-Halogenalkoxy-substituiertes Phenyl-$C_1-C_4$-alkyl steht,
für gegebenenfalls duch Halogen- und $C_1-C_6$-Alkyl-substituiertes Hetaryl steht,
gegebenenfalls für durch Halogen- und $C_1-C_4$-Alkyl-substituiertes Phenoxy-$C_1-C_5$-alkyl steht,

für gegebenfalls durch Halogen, Amino und $C_1-C_4$-Alkyl-substituiertes Hetaryloxy-$C_1$-$C_5$-alkyl steht,

$R^2$ für gegebenenfalls durch Halogen substituiertes: $C_1-C_{16}$-Alkyl, $C_2-C_{16}$-Alkenyl, $C_1$-$C_6$-Alkoxy-$C_2-C_6$-alkyl, $C_1-C_6$-Polyalkoxy-$C_2-C_6$-alkyl steht,

für gegebenenfalls durch Halogen, $C_1-C_4$-Alkyl, $C_1-C_3$-Alkoxy-, $C_1-C_3$-Halogenalkyl-substituiertes Phenyl steht,

sowie die enantiomerenreinen Formen von Verbindungen der Formel (I).

**3.** 3-Aryl-pyrrolidin-2,4-dione der Formel (I) gemäß Anspruch 1, in welcher

X für Methyl, Ethyl, Propyl, i-Propyl, Fluor, Chlor, Brom, Methoxy und Ethoxy steht,

Y für Wasserstoff, Methyl, Ethyl, Propyl, i-Propyl, Butyl, i-Butyl, tert.-Butyl, Chlor, Brom, Methoxy, Ethoxy und Trifluormethyl steht,

Z für Methyl, Ethyl, i-Propyl, Butyl, i-Butyl, tert.-Butyl, Chlor, Brom, Methoxy und Ethoxy steht,

m für eine Zahl von 1-2 steht,

n für eine Zahl von 0-3 steht,

R für Wasserstoff (Ia) oder für die Gruppen der Formeln

$-CO-R^1$ (Ib) ,

$-CO-O-R^2$ (Ic)

oder

A (Id)

steht,

in welcher

A für ein Metallkationäquivalent oder Ammoniumion steht,

$R^1$ für gegebenenfalls durch Fluor oder Chlor substituiertes: $C_1-C_{14}$-Alkyl, $C_2-C_{14}$-Alkenyl, $C_1-C_4$-Alkoxy-$C_1-C_6$-alkyl, $C_1-C_4$-Alkylthio-$C_1-C_6$-alkyl, $C_1-C_4$-Polyalkoxyl-$C_2-C_4$-alkyl und Cycloalkyl mit 3-6 Ringatomen, das durch 1-2 Sauerstoff- und/oder Schwefelatome unterbrochen sein kann steht,

für gegebenfalls durch Fluor-, Chlor, Brom-, Methyl-, Ethyl-, Propyl, i-Propyl, Methoxy, Ethoxy, Trifluormethyl-, Trifluormethoxy-, Nitro- substituiertes Phenyl steht,

für gegebenenfalls durch Fluor-, Chlor-, Brom-, Methyl-, Ethyl-, Propyl-, i-Propyl, Methoxy, Ethoxy, Trifluormethyl, Trifluormethoxy- substituiertes Phenyl-$C_1-C_3$-alkyl steht,

für gegebenenfalls durch Fluor-, Chlor-, Brom-, Methyl-, Ethyl-substituiertes Pyridyl, Pyrimidyl, Thiazolyl und Pyrazolyl steht,

für gegebenenfalls durch Fluor-, Chlor-, Methyl-, Ethyl-substituiertes Phenoxy-$C_1$-$C_4$-alkylsteht,

für gegebenenfalls durch Fluor-, Chlor-, Amino-, Methyl-, Ethyl-, substituiertes Pyridyloxy-$C_1-C_4$-alkyl, Pyrimidyloxy-$C_1-C_4$-alkyl und Thiazolyloxy-$C_1-C_4$-alkyl steht,

$R^2$ für gegebenenfalls durch Fluor oder Chlor substituiertes: $C_1-C_{14}$-Alkyl, $C_2-C_{14}$-Alkenyl, $C_1-C_4$-Alkoxy-$C_2-C_6$-alkyl, $C_1-C_4$-Polyalkoxy-$C_2-C_6$-alkyl steht,

für gegebenenfalls durch Fluor-, Chlor-, Methyl-, Ethyl-, Propyl-, i-Propyl-, Methoxy-, Ethoxy-, Trifluormethyl-, substituiertes Phenyl steht,

sowie die enantiomerenreinen Formen von Verbindungen der Formel (I),

**4.** Verfahren zur Herstellung von 3-Aryl-pyrrolidin-2,4-dion-(e)-Derivaten der Formel (I)

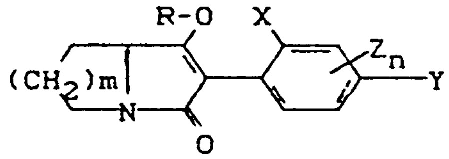

(I)

in welcher

X für $C_1-C_6$-Alkyl, Halogen, $C_1-C_6$-Alkoxy steht,

Y für Wasserstoff, $C_1-C_6$-Alkyl, Halogen, $C_1-C_6$-Alkoxy, $C_1-C_3$-Halogenalkyl steht,

Z für $C_1-C_6$-Alkyl, Halogen, $C_1-C_6$-Alkoxy steht,

m für eine Zahl von 1-4 steht,

n für eine Zahl von 0-3 steht,

R für Wasserstoff (Ia) oder für die Gruppen der Formel

$-CO-R^1$ (Ib) ,
$-CO-O-R^2$ (Ic)

oder

A (Id)

steht,
in welchen

A für ein Metallkationäquivalent oder für ein Ammoniumion steht,

$R^1$ für gegebenenfalls durch Halogen substituiertes: $C_1-C_{20}$-Alkyl, $C_2-C_{20}$-Alkenyl, $C_1-C_8$-Alkoxy-$C_1-C_8$-alkyl, $C_1-C_8$-Alkylthio-$C_1-C_8$-alkyl, $C_1-C_8$-Polyalkoxy-$C_2-C_8$-alkyl und Cycloalkyl mit 3-8 Ringatomen, das durch Sauerstoff und/oder Schwefel unterbrochen sein kann, steht,
für gegebenenfalls durch Halogen-, Nitro-, $C_1-C_6$-Alkyl-, $C_1-C_6$-Alkoxy-, $C_1-C_6$-Halogenalkyl-, $C_1-C_6$-Halogenalkoxy-substituiertes Phenyl;
für gegebenenfalls durch Halogen-, $C_1-C_6$-Alkyl, $C_1-C_6$-Alkoxy-, $C_1-C_6$-Halogenalkyl-, $C_1-C_6$-Halogenalkoxy-substituiertes Phenyl-$C_1-C_6$-alkyl steht,
für gegebenenfalls durch Halogen- und $C_1-C_6$-Alkyl-substituiertes Hetaryl steht,
für gegebenenfalls durch Halogen- und $C_1-C_6$-Alkyl-substituiertes Phenoxy-$C_1-C_6$-alkyl steht,
für gegebenenfalls durch Halogen, Amino und $C_1-C_6$-Alkyl-substituiertes Hetaryloxy-$C_1-C_6$-Alkyl steht,

$R^2$ für gegebenenfalls durch Halogen substituiertes: $C_1-C_{20}$-Alkyl, $C_2-C_{20}$-Alkenyl, $C_1-C_8$-Alkoxy-$C_2-C_8$-alkyl, $C_1-C_8$-Polyalkoxy-$C_2-C_8$-alkyl steht,
für gegebenenfalls durch Halogen-, $C_1-C_6$-Alkyl-, $C_1-C_6$-Alkoxy-, $C_1-C_6$-Halogenalkyl-substituiertes Phenyl steht,

dadurch gekennzeichnet,

(A) daß man zum Erhalt der Verbindungen der Formel (Ia)

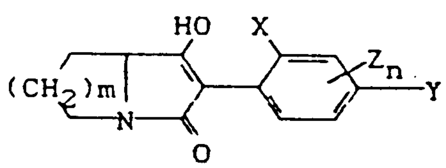

(Ia)

worin

X, Y, Z, m und n die oben angegebene Bedeutung haben,

N-Acylaminosäureester der Formel (II)

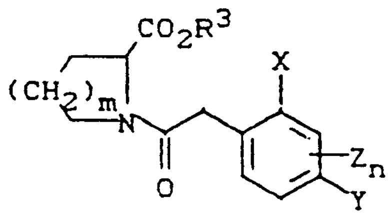

(II)

in welcher
X, Y, Z, m und n die oben angegebene Bedeutung haben
und
$R^3$ für Alkyl steht,
in Gegenwart eines Verdünnungsmittels und in Gegenwart einer Base intramolekular kondensiert,
(B) oder daß man zum Erhalt von Verbindungen der Formel (Ib)

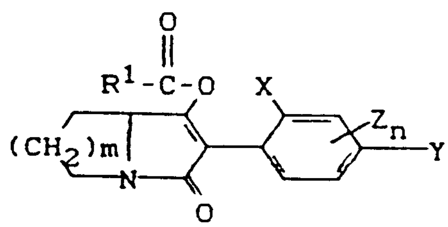

(Ib)

in welcher
$R^1$, X, Y, Z sowie m und n die oben angegebene Bedeutung haben,
Verbindungen der Formel (Ia)

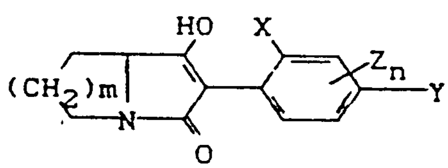

(Ia)

in welcher
X, Y, Z, m und n die oben angegebene Bedeutung haben,
entweder
α) mit Säurehalogeniden der allgemeinen Formel (III)

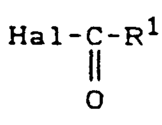

(III)

in welcher
$R^1$ die oben angegebene Bedeutung hat
und
Hal für Halogen, insbesondere Chlor und Brom steht,
gegebenenfalls in Gegenwart eines Verdünnungsmittels und gegebenenfalls in Gegenwart eines Säurebindemittels,
oder
β) mit Carbonsäureanhydriden der allgemeinen Formel(IV)

$R^1-CO-O-CO-R^1$ (IV)

in welcher

$R^1$ die oben angegebene Bedeutung hat,

gegebenenfals in Gegenwart eines Verdünnungsmittels und gegebenenfalls in Gegenwart eines Säurebindemittels umsetzt,

(C) oder daß man zum Erhalt von Verbindungen der Formel (Ic)

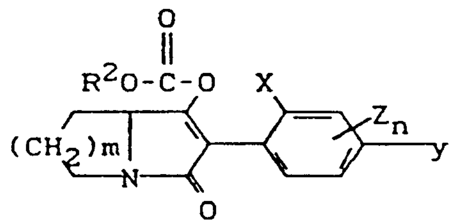

(Ic)

in welcher

$R^2$, X, Y, Z sowie m und n die oben angegebenen Bedeutungen haben, Verbindungen der Formel (Ia)

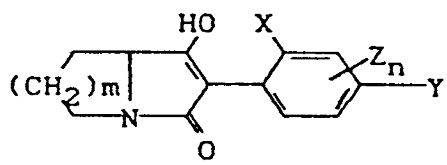

(Ia)

in welcher

X, Y, Z, m und n die oben angegebene Bedeutung haben,

mit Chlorameisensäureestern der allgemeinen Formel (V)

$R^2 - O - CO - Cl$ (V)

in welcher

$R^2$ die oben angegebene Bedeutung hat

gegebenenfalls in Gegenwart eines Verdünnungsmittels und gegebenenfalls in Gegenwart eines Säurebindemittels umsetzt,

(D) oder daß man zum Erhalt von Verbindungen der Formel (Id)

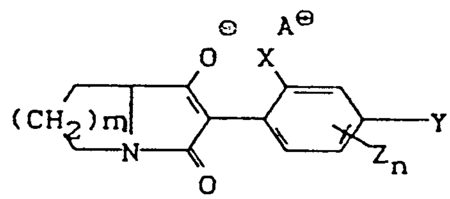

(Id)

in welcher X, Y, Z, A, m und n die oben angegebene Bedeutung haben,

Verbindungen der Formel (Ia)

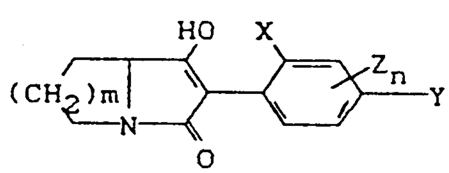

(Ia)

in welcher X, Y, Z, m und n die oben angegebene Bedeutung haben,

mit Metallhydroxiden oder Aminen der allgemeinen Formeln (VIII) und (IX)

$Me_sOH_t$ (VIII) 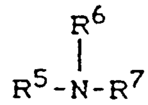 (IX)

in welchen
Me für ein- oder zweiwertige Metallionen
s und t für die Zahl 1 und 2 und
$R^5$, $R^6$, $R^7$ und Y unabhängig voneinander von Wasserstoff und Alkyl stehen,
gegebenenfalls in Gegenwart eines Verdünnungsmittels, umsetzt.

**5.** Insektizide und/oder akarizide und/oder herbizide Mittel, gekennzeichnet durch einen Gehalt an mindestens einem 3-Aryl-pyrrolidin-2,4-dion-Derivat der Formel (I) gemäß Anspruch 1.

**6.** Verwendung von 3-Aryl-pyrrolidin-2,4-dion-Derivaten der Formel (I) gemäß Anspruch 1 zur Bekämpfung von Insekten und/oder Spinnentieren und/oder Unkräutern.

**7.** Verfahren zur Herstellung von insektiziden und/oder akariziden und/oder herbiziden Mitteln, dadurch gekennzeichnet, daß man 3-Aryl-pyrrolidin-2,4-dion-Derivate der Formel (I) mit Streckmitteln und/oder oberflächenaktiven Mitteln vermischt.

**8.** Verwendung von 3-Aryl-pyrrolidin-2,4-dion-Derivaten gemäß Ansprüchen 1 bis 4 zur Herstellung von Mitteln zur Bekämpfung von Mykosen.

**9.** Acylaminosäureester der Formel (II)

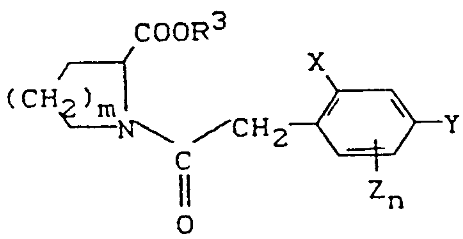

(II)

in welcher
X für $C_1-C_6$-Alkyl, Halogen, $C_1-C_6$-Alkoxy steht,
Y für Wasserstoff, $C_1-C_6$-Alkyl, Halogen, $C_1-C_6$-Alkoxy, $C_1-C_3$-Halogenalkyl steht,
Z für $C_1-C_6$-Alkyl, Halogen, $C_1-C_6$-Alkoxy steht,
m für eine Zahl von 1-4 steht,
n für eine Zahl von 0 - 3 steht,
$R^3$ für $C_{1-6}$-Alkyl steht.

**10.** Verfahren zur Herstellung von Acylaminosäureestern der Formel (II)

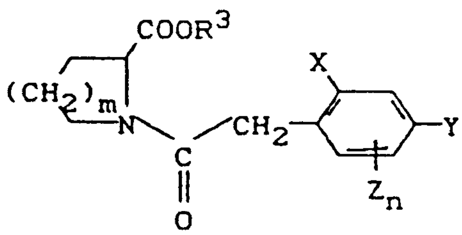

(II)

in welcher
X für $C_1-C_6$-Alkyl, Halogen, $C_1-C_6$-Alkoxy steht,
Y für Wasserstoff, $C_1-C_6$-Alkyl, Halogen, $C_1-C_6$-Alkoxy, $C_1-C_3$-Halogenalkyl steht,

Z für $C_1-C_6$-Alkyl, Halogen, $C_1-C_6$-Alkoxy steht,
m für eine Zahl von 1-4 steht,
n für eine Zahl von 0 - 3 steht,
$R^3$ für $C_{1-6}$-Alkyl steht,

dadurch gekennzeichnet, daß man entweder

a) Aminosäureester der Formel (VI)

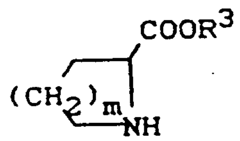

(VI)

in welcher
$R^3$ für Alkyl und
m für die Zahl 1 oder 2 steht,

mit Phenylessigsäurehalogeniden der Formel (VII)

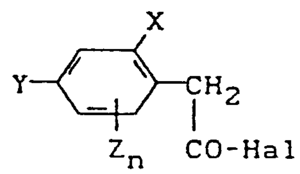

(VII)

in welcher
X, Y, Z und n die oben angegebene Bedeutung haben und
Hal für Chlor oder Brom steht,

acyliert,

oder daß man

b) Acylaminosäuren der Formel (IIa),

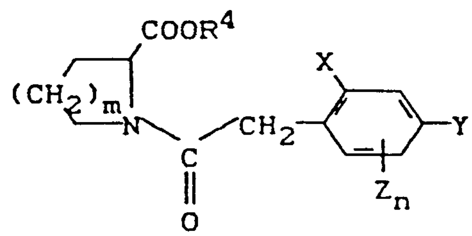

(IIa)

in welcher
X, Y, Z, m und n die oben angegebene Bedeutung haben und
$R^4$ für Wasserstoff steht,

verestert.

## Claims

**1.** 3-Aryl-pyrrolidine-2,4-dione(s) of the formula (I)

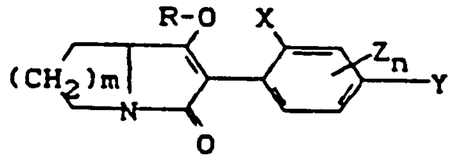

(I)

in which

X represents $C_1-C_6$-alkyl, halogen and $C_1-C_6$-alkoxy,

Y represents hydrogen, $C_1-C_6$-alkyl, halogen, $C_1-C_6$-alkoxy and $C_1-C_3$-halogenoalkyl,

Z represents $C_1-C_6$-alkyl, halogen and $C_1-C_6$-alkoxy,

m represents a number from 1-4,

n represents a number from 0-3,

R represents hydrogen (Ia), or represents the groups of the formula

$-CO-R^1$ (Ib) ,
$-CO-O-R^2$ (Ic)

or

A (Id)

in which

A represents a metal cation equivalent or represents an ammonium ion,

$R^1$ represents optionally halogen-substituted: $C_1-C_{20}$-alkyl, $C_2-C_{20}$-alkenyl, $C_1-C_8$-alkoxy-$C_1-C_8$-alkyl, $C_1-C_8$-alkylthio-$C_1-C_8$-alkyl, $C_1-C_8$-polyalkoxy-$C_2-C_8$-alkyl and cycloalkyl which has 3-8 ring atoms and which can be interrupted by oxygen and/or sulphur,
or represents optionally halogen-, nitro-, $C_1-C_6$-alkyl-, $C_1-C_6$-alkoxy-, $C_1-C_6$-halogenoalkyl-, or $C_1-C_6$-halogenoalkoxy-substituted phenyl;
or represents optionally halogen-, $C_1-C_6$-alkyl-, $C_1-C_6$-alkoxy-, $C_1-C_6$-halogenoalkyl- or $C_1-C_6$-halogenoalkoxy-substituted phenyl-$C_1-C_6$-alkyl,
or represents optionally halogen- and $C_1-C_6$-alkyl-substituted hetaryl,
or represents optionally halogen- and $C_1-C_6$-alkyl-substituted phenoxy-$C_1-C_6$-alkyl,
or represents optionally halogen-, amino- and $C_1-C_6$-alkyl-substituted hetaryloxy-$C_1-C_6$-alkyl,

$R^2$ represents optionally halogen-substituted: $C_1-C_{20}$-alkyl, $C_2-C_{20}$-alkenyl, $C_1-C_8$-alkoxy-$C_2-C_8$-alkyl and $C_1-C_8$-polyalkoxy-$C_2-C_8$-alkyl,
or represents optionally halogen-, $C_1-C_6$-alkyl-, $C_1-C_6$-alkoxy-and $C_1-C_6$-halogenoalkyl-substituted phenyl,

and the enantiomeric forms of compounds of the formula (I).

**2.** 3-Aryl-pyrrolidine-2,4-diones of the formula (I) according to Claim 1,
in which

X represents $C_1-C_4$-alkyl, halogen and $C_1-C_4$-alkoxy,

Y represents hydrogen, $C_1-C_6$-alkyl, halogen, $C_1-C_4$-alkoxy and $C_1-C_2$-halogenoalkyl,

Z represents $C_1-C_4$-alkyl, halogen and $C_1-C_4$-alkoxy,

m represents a number from 1-3,

n represents a number from 0-3,

R represents hydrogen (Ia), or represents the groups of the formula

$-CO-R^1$ (Ib) ,
$-CO-O-R^2$ (Ic)

or

A (Id)

in which

A represents a metal cation equivalent or represents an ammonium ion,

$R^1$ represents optionally halogen-substituted: $C_1-C_{16}$-alkyl, $C_2-C_{16}$-alkenyl, $C_1-C_6$-alkoxy-$C_1-C_6$-alkyl, $C_1-C_6$-alkylthio-$C_1-C_6$-alkyl, $C_1-C_6$-polyalkoxy-$C_2-C_6$-alkyl and cycloalkyl which has 3-7 ring atoms and which can be interrupted by 1-2 oxygen and/or sulphur atoms,
or represents optionally halogen-, nitro-, $C_1-C_4$-alkyl, $C_1-C_4$-alkoxy-, $C_1-C_3$-halogenoalkyl- or $C_1-C_3$-halogenoalkoxy-substituted phenyl,
or represents optionally halogen-, $C_1-C_4$-alkyl-, $C_1-C_4$-alkoxy-, $C_1-C_3$-halogenoalkyl- or $C_1-C_3$-halogenoalkoxy-substituted phenyl-$C_1-C_4$-alkyl,
or represents optionally halogen- and $C_1-C_6$-alkyl-substituted hetaryl,
or represents optionally halogen- and $C_1-C_4$-alkyl-substituted phenoxy-$C_1-C_5$-alkyl,
or represents optionally halogen-, amino- and $C_1-C_4$-alkyl-substituted hetaryloxy-$C_1-C_5$-alkyl,

$R^2$ represents optionally halogen-substituted: $C_1-C_{16}$-alkyl, $C_2-C_{16}$-alkenyl, $C_1-C_6$-alkoxy-$C_2-C_6$-alkyl and $C_1-C_6$-polyalkoxy-$C_2-C_6$-alkyl,
or represents optionally halogen-, $C_1-C_4$-alkyl-, $C_1-C_3$-alkoxy- or $C_1-C_3$-halogenoalkyl-substituted phenyl,

and the enantiomerically pure forms of compounds of the formula (I).

**3.** 3-Aryl-pyrrolidine-2,4-diones of the formula (I) according to Claim 1,
in which

X represents methyl, ethyl, propyl, i-propyl, fluorine, chlorine, bromine, methoxy and ethoxy,

Y represents hydrogen, methyl, ethyl, propyl, i-propyl, butyl, i-butyl, tert.-butyl, chlorine, bromine, methoxy, ethoxy and trifluoromethyl,

Z represents methyl, ethyl, i-propyl, butyl, i-butyl, tert.-butyl, chlorine, bromine, methoxy and ethoxy,

m represents a number from 1-2,

n represents a number from 0-3,

R represents hydrogen (Ia) or represents the groups of the formulae

$-CO-R^1$ (Ib) ,
$-CO-O-R^2$ (Ic)

or

A (Id)

in which

A represents a metal cation equivalent or an ammonium ion,

$R^1$ represents optionally fluorine- or chlorine-substituted: $C_1-C_{14}$-alkyl, $C_2-C_{14}$-alkenyl, $C_1-C_4$-alkoxy-$C_1-C_6$-alkyl, $C_1-C_4$-alkylthio-$C_1-C_6$-alkyl, $C_1-C_4$-polyalkoxyl-$C_2-C_4$-alkyl and cycloalkyl which has 3-6 ring atoms and which can be interrupted by 1-2 oxygen and/or sulphur atoms,
or represents optionally fluorine-, chlorine-, bromine-, methyl-, ethyl-, propyl-, i-propyl-, methoxy-, ethoxy-, trifluoromethyl-, trifluoromethoxy- or nitro-substituted phenyl,
or represents optionally fluorine-, chlorine-, bromine-, methyl-, ethyl-, propyl-, i-propyl-, methoxy-, ethoxy-, trifluoromethyl- or trifluoromethoxy-substituted phenyl-$C_1-C_3$-alkyl,
or represents optionally fluorine-, chlorine-, bromine-, methyl- or ethyl-substituted pyridyl, pyrimidyl, thiazolyl and pyrazolyl,
or represents optionally fluorine-, chlorine-, methyl- or ethyl-substituted phenoxy-$C_1-C_4$-alkyl,

or represents optionally fluorine-, chlorine-, amino-, methyl- or ethyl-substituted pyridyloxy-$C_1$-$C_4$-alkyl, pyrimidyloxy-$C_1$-$C_4$-alkyl and thiazolyloxy-$C_1$-$C_4$-alkyl,

$R^2$ represents optionally fluorine- or chlorine-substituted: $C_1$-$C_{14}$-alkyl, $C_2$-$C_{14}$-alkenyl, $C_1$-$C_4$-alkoxy-$C_2$-$C_6$-alkyl and $C_1$-$C_4$-polyalkoxy-$C_2$-$C_6$-alkyl,
or represents optionally fluorine-, chlorine-, methyl-, ethyl-, propyl-, i-propyl, methoxy-, ethoxy- or trifluoromethyl-substituted phenyl,

and the enantiomerically pure forms of compounds of the formula (I).

**4.** Process for the preparation of 3-aryl-pyrrolidine-2,4-dione(s) derivatives of the formula (I)

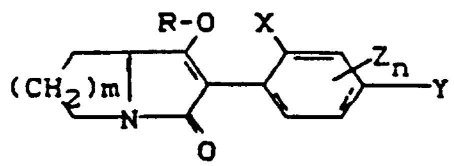

(I)

in which

X represents $C_1$-$C_6$-alkyl, halogen and $C_1$-$C_6$-alkoxy,

Y represents hydrogen, $C_1$-$C_6$-alkyl, halogen, $C_1$-$C_6$-alkoxy and $C_1$-$C_3$-halogenoalkyl,

Z represents $C_1$-$C_6$-alkyl, halogen and $C_1$-$C_6$-alkoxy,

m represents a number from 1-4,

n represents a number from 0-3,

R represents hydrogen (Ia), or represents the groups of the formula

$-CO-R^1$ (Ib) ,
$-CO-O-R^2$ (Ic)

or

A (Id)

in which

A represents a metal cation equivalent or represents an ammonium ion,

$R^1$ represents optionally halogen-substituted: $C_1$-$C_{20}$-alkyl, $C_2$-$C_{20}$-alkenyl, $C_1$-$C_8$-alkoxy-$C_1$-$C_8$-alkyl, $C_1$-$C_8$-alkylthio-$C_1$-$C_8$-alkyl, $C_1$-$C_8$-polyalkoxy-$C_2$-$C_8$-alkyl and cycloalkyl which has 3-8 ring atoms and which can be interrupted by oxygen and/or sulphur,
or represents optionally halogen-, nitro-, $C_1$-$C_6$-alkyl, $C_1$-$C_6$-alkoxy-, $C_1$-$C_6$-halogenoalkyl- or $C_1$-$C_6$-halogenoalkoxy-substituted phenyl;
or represents optionally halogen-, $C_1$-$C_6$-alkyl-, $C_1$-$C_6$-alkoxy-, $C_1$-$C_6$-halogenoalkyl- or $C_1$-$C_6$-halogenoalkoxy-substituted phenyl-$C_1$-$C_6$-alkyl,
or represents optionally halogen- and $C_1$-$C_6$-alkyl-substituted hetaryl,
or represents optionally halogen- and $C_1$-$C_6$-alkyl-substituted phenoxy-$C_1$-$C_6$-alkyl,
or represents optionally halogen, amino- and $C_1$-$C_6$-alkyl-substituted hetaryloxy-$C_1$-$C_6$-alkyl,

$R^2$ represents optionally halogen-substituted: $C_1$-$C_{20}$-alkyl, $C_2$-$C_{20}$-alkenyl, $C_1$-$C_8$-alkoxy-$C_2$-$C_8$-alkyl and $C_1$-$C_8$-polyalkoxy-$C_2$-$C_8$-alkyl,
or represents optionally halogen-, $C_1$-$C_6$-alkyl-, $C_1$-$C_6$-alkoxy-and $C_1$-$C_6$-halogenoalkyl-substituted phenyl,

characterized in that,

(A) in order to obtain the compounds of the formula (Ia),

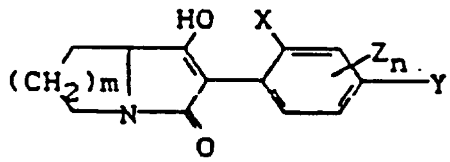

(Ia)

where
X, Y, Z, m and n have the abovementioned meaning,
N-acylamino acid esters of the formula (II)

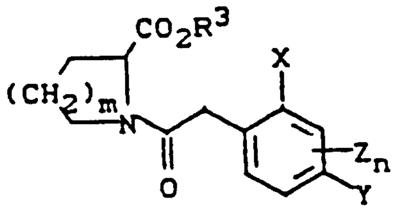

(II)

in which
X, Y, Z, m and n have the abovementioned meaning
and
$R^3$ represents alkyl,
are subjected to an intramolecular condensation reaction in the presence of a diluent and in the presence of a base,
(B) or, in order to obtain compounds of the formula (Ib),

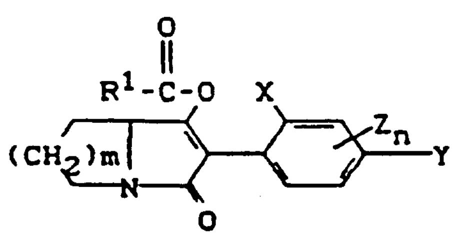

(Ib)

in which
$R^1$, X, Y, Z as well as m and n have the abovementioned meaning,
compounds of the formula (Ia)

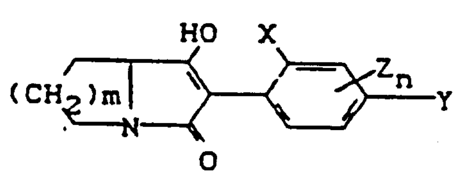

(Ia)

in which
X, Y, Z, m and n have the abovementioned meaning,
are reacted either

α) with acid halides of the general formula (III)

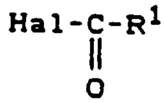

(III)

in which
R[1] has the abovementioned meaning and
Hal represents halogen, in particular chlorine and bromine,
if appropriate in the presence of a diluent and if appropriate in the presence of an acid-binding agent,
or
β) with carboxylic anhydrides of the general formula (IV)

$R^1-CO-O-CO-R^1$ (IV)

in which
R[1] has the abovementioned meaning,
if appropriate in the presence of a diluent and if appropriate in the presence of an acid-binding agent,
(C) or, in order to obtain compounds of the formula (Ic)

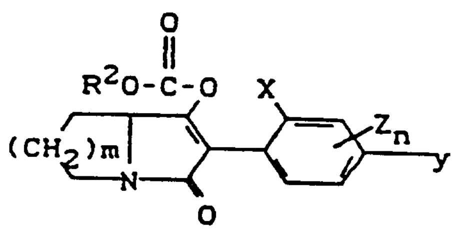

(Ic)

in which
$R^2$, X, Y, Z as well as m and n have the abovementioned meanings, compounds of the formula (Ia)

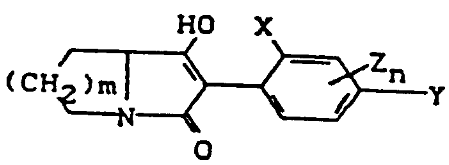

(Ia)

in which
X, Y, Z, m and n have the abovementioned meaning,
are reacted with chloroformic acid esters of the general formula (V)

$R^2-O-CO-Cl$ (V)

in which
$R^2$ has the abovementioned meaning,
if appropriate in the presence of a diluent and if appropriate in the presence of an acid-binding agent,

(D) or, in order to obtain compounds of the formula (Id)

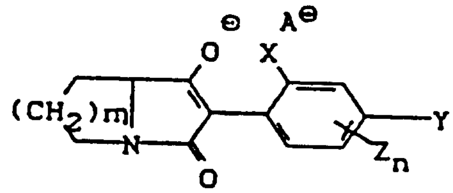

(Id)

in which X, Y, Z, A, m and n have the abovementioned meaning, compounds of the formula (Ia)

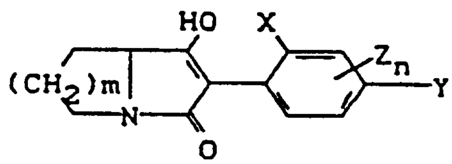

(Ia)

in which X, Y, Z, m and n have the abovementioned meaning, are reacted with metal hydroxides or amines of the general formulae (VIII) and (IX)

$Me_sOH_t$ (VIII)

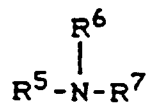

(IX)

in which
Me represents monovalent or divalent metal ions,
s and t represent the number 1 and 2 and
$R^5$, $R^6$, $R^7$ and Y independently of one another represent hydrogen and alkyl,
if appropriate in the presence of a diluent.

**5.** Insecticidal and/or acaricidal and/or herbicidal agents, characterised in that they contain at least one 3-aryl-pyrrolidine-2,4-dione derivative of the formula (I) according to Claim 1.

**6.** Use of 3-aryl-pyrrolidine-2,4-dione derivatives of the formula (I) according to Claim 1 for combating insects and/or arachnids and/or weeds.

**7.** Process for the preparation of insecticidal and/or acaricidal and/or herbicidal agents, characterised in that 3-aryl-pyrrolidine-2,4-dione derivatives of the formula (I) are mixed with extenders and/or surface-active agents.

**8.** Use of 3-aryl-pyrrolidine-2,4-dione derivatives according to Claims 1 to 4 for the preparation of agents for combating mycoses.

**9.** Acylamino acid esters of the formula (II)

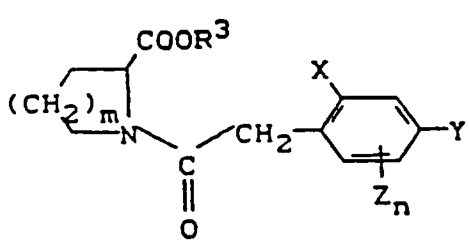

(II)

in which

X represents $C_1-C_6$-alkyl, halogen and $C_1-C_6$-alkoxy,
Y represents hydrogen, $C_1-C_6$-alkyl, halogen, $C_1-C_6$-alkoxy and $C_1-C_3$-halogenoalkyl,
Z represents $C_1-C_6$-alkyl, halogen and $C_1-C_6$-alkoxy,
m represents a number from 1 to 4,
n represents a number from 0 to 3 and
$R^3$ represents $C_{1-6}$-alkyl.

**10.** Process for the preparation of acylamino acid esters of the formula (II)

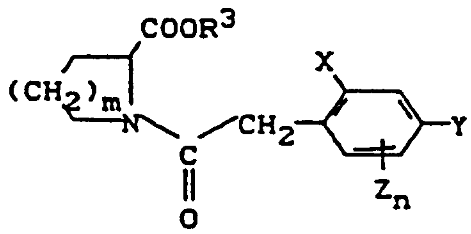

(II)

in which

X represents $C_1-C_6$-alkyl, halogen and $C_1-C_6$-alkoxy,
Y represents hydrogen, $C_1-C_6$-alkyl, halogen, $C_1-C_6$-alkoxy and $C_1-C_3$-halogenoalkyl,
Z represents $C_1-C_6$-alkyl, halogen and $C_1-C_6$-alkoxy,
m represents a number from 1 to 4,
n represents a number from 0 to 3 and
$R^3$ represents $C_{1-6}$-alkyl,

characterised in that either

a) amino acid esters of the formula (VI)

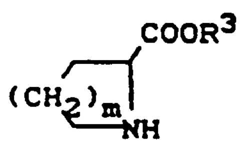

(VI)

in which

$R^3$ represents alkyl and
m represents the number 1 or 2,

are acylated with phenylacetic acid halides of the formula (VII)

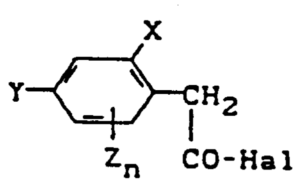

(VII)

in which

X, Y, Z and n have the abovementioned meaning and
Hal represents chlorine or bromine,

or in that

b) acylamino acids of the formula (IIa),

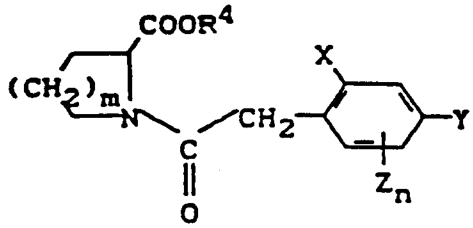

(IIa)

in which
X, Y, Z, m and n have the abovementioned meaning
and
$R^4$ represents hydrogen,
are esterified.

## Revendications

**1.** 3-aryl-pyrrolidine-2,4-diones ou leurs dérivés, de formule

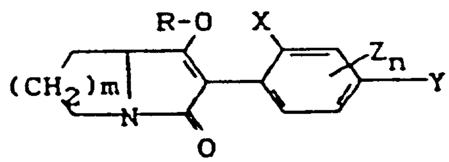

(I)

dans laquelle

X est un groupe alkyle en $C_1$ à $C_6$, un halogène, un groupe alkoxy en $C_1$ à $C_6$,
Y représente l'hydrogène, un groupe alkyle en $C_1$ à $C_6$, un halogène, un groupe alkoxy en $C_1$ à $C_6$, un groupe halogénalkyle en $C_1$ à $C_3$,
Z est un groupe alkyle en $C_1$ à $C_6$, un halogène, un groupe alkoxy en $C_1$ à $C_6$,
m est un nombre de 1 à 4,
n est un nombre de 0 à 3,
R représente l'hydrogène (Ia) ou les groupes de formule

$-CO-R^1$ (Ib),
$-CO-O-R^2$ (Ic)

ou

A (Id)

dans lesquels

A représente un équivalent de cation métallique ou un ion ammonium,
$R^1$ est un groupe, éventuellement substitué par un halogène, alkyle en $C_1$ à $C_{20}$, alcényle en $C_2$ à $C_{20}$, (alkoxy en $C_1$ à $C_8$)-alkyle en $C_1$ à $C_8$, (alkylthio en $C_1$ à $C_8$)-(alkyle en $C_1$ à $C_8$), (polyalkoxy en $C_1$ à $C_8$)-(alkyle en $C_2$ à $C_8$) et cycloalkyle ayant 3 à 8 atomes dans le noyau, qui peut être interrompu par de l'oxygène et/ou du soufre,
un groupe phényle éventuellement substitué par un radical halogéno, nitro, alkyle en $C_1$ à $C_6$, alkoxy en $C_1$ à $C_6$, halogénalkyle en $C_1$ à $C_6$, halogénalkoxy en $C_1$ à $C_6$ ;
un groupe phényl-(alkyle en $C_1$ à $C_6$) éventuellement substitué par un radical halogéno, alkyle en $C_1$ à $C_6$, alkoxy en $C_1$ à $C_6$, halogénalkyle en $C_1$ à $C_6$, halogénalkoxy en $C_1$ à $C_6$,
un groupe hétaryle portant éventuellement un substituant halogéno et alkyle en $C_1$ à $C_6$,
un groupe phénoxy-(alkyle en $C_1$ à $C_6$) portant éventuellement un substituant halogéno et alkyle en $C_1$ à $C_6$,
un groupe hétaryloxy-(alkyle en $C_1$ à $C_6$) portant éventuellement un substituant halogéno,

amino et alkyle en $C_1$ à $C_6$,

$R^2$ est un groupe, éventuellement substitué par un halogène : alkyle en $C_1$ à $C_{20}$, alcényle en $C_2$ à $C_{20}$, (alkoxy en $C_1$ à $C_8$) – (alkyle en $C_2$ à $C_8$), (polyalkoxy en $C_1$ à $C_8$) – (alkyle en $C_2$ à $C_8$),
un groupe phényle portant éventuellement un substituant halogéno, alkyle en $C_1$ à $C_6$, alkoxy en $C_1$ à $C_6$, halogénalkyle en $C_1$ à $C_6$,

ainsi que les formes énantiomériques de composés de formule (I).

**2.** 3 – aryl – pyrrolidine – 2,4 – diones de formule (I) suivant la revendication 1, dans laquelle

X est un groupe alkyle en $C_1$ à $C_4$, un halogène, un groupe alkoxy en $C_1$ à $C_4$,

Y est l'hydrogène, un groupe alkyle en $C_1$ à $C_6$, un halogène, un groupe alkoxy en $C_1$ à $C_4$, un groupe halogénalkyle en $C_1$ ou $C_2$,

Z est un groupe alkyle en $C_1$ à $C_4$, un halogène, un groupe alkoxy en $C_1$ à $C_4$,

m est un nombre de 1 à 3,

n est un nombre de 0 à 3,

R représente l'hydrogène (Ia) ou les groupes de formule

$-CO-R^1$ (Ib) ,
$-CO-O-R^2$ (Ic)

ou

A (Id)

dans lesquels,

A représente un équivalent de cation métallique ou un ion ammonium,

$R^1$ est un groupe, éventuellement substitué par un halogène :
alkyle en $C_1$ à $C_{16}$, alcényle en $C_2$ à $C_{16}$, (alkoxy en $C_1$ à $C_6$) – (alkyle en $C_1$ à $C_6$), (alkylthio en $C_1$ à $C_6$) – (alkyle en $C_1$ à $C_6$), (polyalkoxy en $C_1$ à $C_6$) – (alkyle en $C_2$ à $C_6$) et cycloalkyle ayant 3 à 7 atomes dans le noyau qui peut être interrompu par 1 – 2 atomes d'oxygène et/ou de soufre,
un groupe phényle portant éventuellement un substituant halogéno, nitro, alkyle en $C_1$ à $C_4$, alkoxy en $C_1$ à $C_4$, halogénalkyle en $C_1$ à $C_3$, halogénalkoxy en $C_1$ à $C_3$,
un groupe phényl – (alkyle en $C_1$ à $C_4$) portant éventuellement un substituant halogéno, alkyle en $C_1$ à $C_4$, alkoxy en $C_1$ à $C_4$, halogénalkyle en $C_1$ à $C_3$, halogénalkoxy en $C_1$ à $C_3$,
un groupe hétaryle portant éventuellement un substituant halogéno et alkyle en $C_1$ à $C_6$,
un groupe phénoxy – (alkyle en $C_1$ à $C_5$) portant éventuellement un substituant halogéno et alkyle en $C_1$ à $C_4$,
un groupe hétaryloxy – (alkyle en $C_1$ à $C_5$) portant éventuellement un substituant halogéno, amino, et alkyle en $C_1$ à $C_4$,

$R^2$ est un groupe, éventuellement substitué par un halogène : alkyle en $C_1$ à $C_{16}$, alcényle en $C_2$ à $C_{16}$, (alkoxy en $C_1$ à $C_6$) – (alkyle en $C_2$ à $C_6$), (polyalkoxy en $C_1$ à $C_6$) – (alkyle en $C_2$ à $C_6$),
un groupe phényle portant éventuellement un substituant halogéno, alkyle en $C_1$ à $C_4$, alkoxy en $C_1$ à $C_3$, halogénalkyle en $C_1$ à $C_3$,

ainsi que les formes de pureté énantiomérique de composés de formule (I).

**3.** 3 – aryl – pyrrolidine – 2,4 – diones de formule (I) suivant la revendication 1, dans laquelle

X représente les radicaux méthyle, éthyle, propyle, isopropyle, fluoro, chloro, bromo, méthoxy et éthoxy,

Y représente l'hydrogène, les radicaux méthyle, éthyle, propyle, isopropyle, butyle, isobutyle, tertio – butyle, chloro, bromo, méthoxy, éthoxy, et trifluorométhyle,

Z représente les radicaux méthyle, éthyle, isopropyle, butyle, isobutyle, tertio – butyle, chloro, bromo, méthoxy et éthoxy,

m est un nombre de valeur 1 – 2,

n est un nombre de valeur 0 – 3,

R représente l'hydrogène (Ia) ou les groupes de formules

$-CO-R^1$ (Ib) ,
$-CO-O-R^2$ (Ic)

ou

A (Id)

où

A est un équivalent de cation métallique ou un ion ammonium,

$R^1$ est un groupe, éventuellement substitué par du fluor ou du chlore : alkyle en $C_1$ à $C_{14}$, alcényle en $C_2$ à $C_{14}$, (alkoxy en $C_1$ à $C_4$) (alkyle en $C_1$ à $C_6$), (alkylthio en $C_1$ à $C_4$)-(alkyle en $C_1$ à $C_6$), (polyalkoxy en $C_1$ à $C_4$)-alkyle en $C_2$ à $C_4$, et cycloalkyle ayant 3 à 6 atomes dans le noyau, qui peut être interrompu par 1-2 atomes d'oxygène et/ou de soufre,
un groupe phényle portant éventuellement un substituant fluoro, chloro, bromo, méthyle, éthyle, propyle, isopropyle, méthoxy, éthoxy, trifluorométhyle, trifluorométhoxy, nitro,
un groupe phényl- (alkyle en $C_1$ à $C_3$) portant éventuellement un substituant fluoro, chloro, bromo, méthyle, éthyle, propyle, isopropyle, méthoxy, éthoxy, trifluorométhyle, trifluorométhoxy,
un groupe pyridyle, pyrimidyle, thiazolyle et pyrazolyle portant éventuellement un substituant fluoro, chloro, bromo, méthyle, éthyle,
un groupe phénoxy-(alkyle en $C_1$ à $C_4$) portant éventuellement un substituant fluoro, chloro, méthyle, éthyle,
un groupe pyridyloxy-(alkyle en $C_1$ à $C_4$), pyrimidyloxy-(alkyle en $C_1$ à $C_4$) et un groupe thiazolyloxy-(alkyle en $C_1$ à $C_4$) portant éventuellement un substituant fluoro, chloro, amino, méthyle, éthyle,

$R^2$ est un groupe éventuellement substitué par du fluor ou du chlore : alkyle en $C_1$ à $C_{14}$, alcényle en $C_2$ à $C_{14}$, (alkoxy en $C_1$ à $C_4$)-(alkyle en $C_2$ à $C_6$), (polyalkoxy en $C_1$ à $C_4$)-(alkyle en $C_2$ à $C_6$),
un groupe phényle portant éventuellement un substituant fluoro, chloro, méthyle, éthyle, propyle, isopropyle, méthoxy, éthoxy, trifluorométhyle,

ainsi que les formes de pureté énantiomérique de composés de formule (I).

**4.** Procédé de production de 3-aryl-pyrrolidine-2,4-diones ou de leurs dérivés de formule (I)

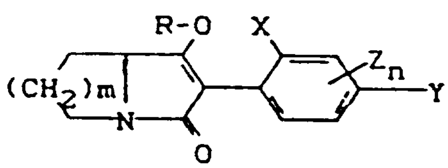

(I)

dans laquelle

X est un groupe alkyle en $C_1$ à $C_6$, un halogène, un groupe alkoxy en $C_1$ à $C_6$,

Y représente l'hydrogène, un groupe alkyle en $C_1$ à $C_6$, un halogène, un groupe alkoxy en $C_1$ à $C_6$, un groupe halogénalkyle en $C_1$ à $C_3$,

Z est un groupe alkyle en $C_1$ à $C_6$, un halogène, un groupe alkoxy en $C_1$ à $C_6$,

m est un nombre de 1 à 4,

n est un nombre de 0 à 3,

R représente l'hydrogène (Ia) ou les groupes de formule

$-CO-R^1$ (Ib) ,
$-CO-O-R^2$ (Ic)

ou

A (Id)

dans lesquels

A représente un équivalent de cation métallique ou un ion ammonium,

$R^1$ est un groupe, éventuellement substitué par un halogène, alkyle en $C_1$ à $C_{20}$, alcényle en $C_2$ à $C_{20}$, (alkoxy en $C_1$ à $C_8$)-alkyle en $C_1$ à $C_8$, (alkylthio en $C_1$ à $C_8$)-(alkyle en $C_1$ à $C_8$), (polyalkoxy en $C_1$ à $C_8$)-(alkyle en $C_2$ à $C_8$) et cycloalkyle ayant 3 à 8 atomes dans le noyau, qui peut être interrompu par de l'oxygène et/ou du soufre,

un groupe phényle éventuellement substitué par un radical halogéno, nitro, alkyle en $C_1$ à $C_6$, alkoxy en $C_1$ à $C_6$, halogénalkyle en $C_1$ à $C_6$, halogénalkoxy en $C_1$ à $C_6$ ;

un groupe phényl-(alkyle en $C_1$ à $C_6$) éventuellement substitué par un radical halogéno, alkyle en $C_1$ à $C_6$, alkoxy en $C_1$ à $C_6$, halogénalkyle en $C_1$ à $C_6$, halogénalkoxy en $C_1$ à $C_6$,

un groupe hétaryle portant éventuellement un substituant halogéno et alkyle en $C_1$ à $C_6$,

un groupe phénoxy-(alkyle en $C_1$ à $C_6$) portant éventuellement un substituant halogéno et alkyle en $C_1$ à $C_6$,

un groupe hétaryloxy-(alkyle en $C_1$ à $C_6$) portant éventuellement un substituant halogéno, amino et alkyle en $C_1$ à $C_6$,

$R^2$ est un groupe éventuellement substitué par un halogène : alkyle en $C_1$ à $C_{20}$, alcényle en $C_2$ à $C_{20}$, (alkoxy en $C_1$ à $C_8$)-(alkyle en $C_2$ à $C_8$), (polyalkoxy en $C_1$ à $C_8$)-(alkyle en $C_2$ à $C_8$),

un groupe phényle portant éventuellement un substituant halogéno, alkyle en $C_1$ à $C_6$, alkoxy en $C_1$ à $C_6$, halogénalkyle en $C_1$ à $C_6$,

caractérisé en ce que

(A) pour obtenir les composés de formule (Ia)

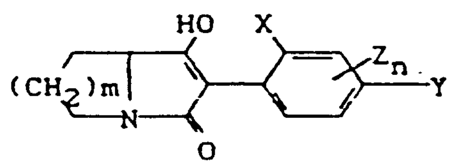

(Ia)

dans laquelle

X, Y, Z, m et n ont la définition indiquée ci-dessus,

on effectue la condensation intramoléculaire d'esters de N-acylaminoacides de formule (II)

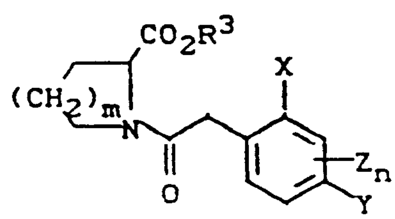

(II)

dans laquelle

X, Y, Z, m et n ont la définition indiquée ci-dessus et

$R^3$ est un groupe alkyle,

en présence d'un diluant et en présence d'une base, ou bien

(B) en vue d'obtenir des composés de formule (Ib)

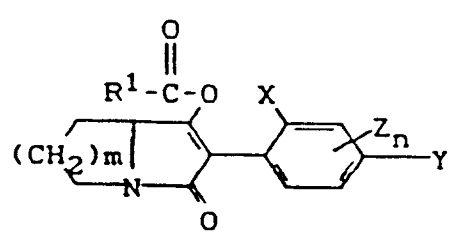

(Ib)

dans laquelle

$R^1$, X, Y, Z ainsi que m et n ont la définition indiquée ci-dessus, on fait réagir des composés de formule (Ia)

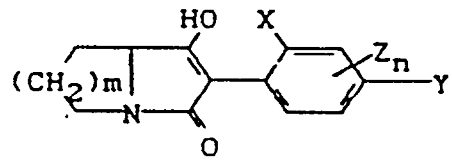

(Ia)

dans laquelle

X, Y, Z, m et n ont la définition indiquée ci-dessus,

ou bien

α) avec des halogénures d'acides de formule générale (III)

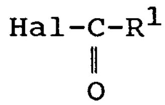

(III)

dans laquelle

$R^1$ a la définition indiquée ci-dessus et

Hal est un halogène, notamment le chlore et le brome,

le cas échéant en présence d'un diluant et en la présence éventuelle d'un accepteur d'acide,

ou bien

β) avec des anhydrides d'acides carboxyliques de formule générale (IV)

$R^1-CO-O-CO-R^1$ (IV)

dans laquelle

$R^1$ a la définition indiquée ci-dessus,

le cas échéant en présence d'un diluant et en la présence éventuelle d'un accepteur d'acide, ou bien

C) en vue d'obtenir des composés de formule (Ic)

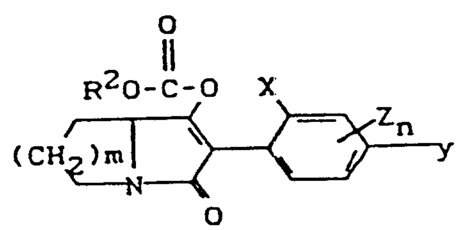

(Ic)

dans laquelle

$R^2$, X, Y, Z ainsi que m et n ont les définitions indiquées ci-dessus, on fait réagir des composés de formule (Ia)

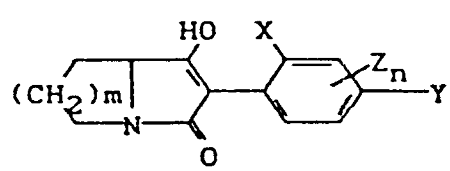

(Ia)

dans laquelle
X, Y, Z, m et n ont la définition indiquée ci-dessus,
avec des esters d'acide chloroformique de formule générale (V)

$R^2 - O - CO - Cl$ (V)

dans laquelle
$R^2$ a la définition indiquée ci-dessus,
le cas échéant en présence d'un diluant et en la présence éventuelle d'un accepteur d'acide, ou bien
(D) en vue d'obtenir des composés de formule (Id)

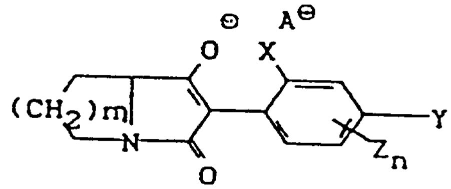

(Id)

dans laquelle X, Y, Z, A, m et n ont la définition indiquée ci-dessus,
on fait réagir des composés de formule (Ia)

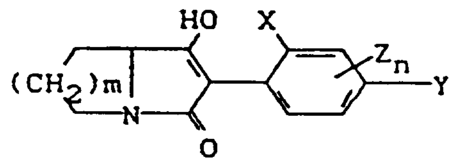

(Ia)

dans laquelle X, Y, Z, m et n ont la définition indiquée ci-dessus,
avec des hydroxydes de métaux ou des amines de formules générales (VIII) et (IX)

$Me_sOH_t$ (VIII)

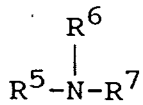

(IX)

dans lesquelles

| | |
|---|---|
| Me | représente un ion métallique monovalent ou divalent |
| s et t | représentent les nombres 1 et 2 et |
| $R^5$, $R^6$, $R^7$ et Y | représentent indépendamment les uns des autres l'hydrogène et un groupe alkyle, éventuellement en présence d'un diluant. |

**5.** Compositions insecticides et/ou acaracides et/ou herbicides, caractérisées par une teneur en au moins un dérivé de 3-aryl-pyrrolidine-2,4-dione de formule (I) suivant la revendication 1.

**6.** Utilisation de dérivés de 3-aryl-pyrrolidine-2,4-diones de formule (I) suivant la revendication 1 pour combattre des insectes et/ou des acariens et/ou des mauvaises herbes.

**7.** Procédé de préparation de compositions insecticides et/ou acaricides et/ou herbicides, caractérisé en ce qu'on mélange des dérivés de 3-aryl-pyrrolidine-2,4-diones de formule (I) avec des diluants et/ou des agents tensio-actifs.

**8.** Utilisation de dérivés de 3-aryl-pyrrolidine-2,4-diones suivant les revendications 1 à 4 pour la préparation de compositions destinées à combattre des mycoses.

**9.** Esters d'acylaminoacides de formule (II)

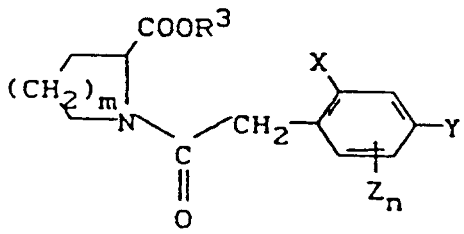

(II)

dans laquelle

X est un groupe alkyle en $C_1$ à $C_6$, un halogène, un groupe alkoxy en $C_1$ à $C_6$,

Y représente l'hydrogène, un groupe alkyle en $C_1$ à $C_6$, un halogène, un groupe alkoxy en $C_1$ à $C_6$, un groupe halogénalkyle en $C_1$ à $C_3$,

Z est un groupe alkyle en $C_1$ à $C_6$, un halogène, un groupe alkoxy en $C_1$ à $C_6$,

m est un nombre de 1 à 4,

n est un nombre de 0 à 3,

$R^3$ est un groupe alkyle en $C_1$ à $C_6$.

**10.** Procédé de production d'esters d'acylaminoacides de formule (II)

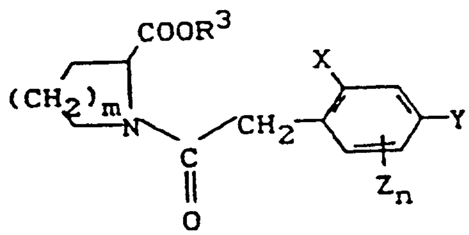

(II)

dans laquelle

X est un groupe alkyle en $C_1$ à $C_6$, un halogène, un groupe alkoxy en $C_1$ à $C_6$,

Y représente l'hydrogène, un groupe alkyle en $C_1$ à $C_6$, un halogène, un groupe alkoxy en $C_1$ à $C_6$, un groupe halogénalkyle en $C_1$ à $C_3$,

Z est un groupe alkyle en $C_1$ à $C_6$, un halogène, un groupe alkoxy en $C_1$ à $C_6$,

m est un nombre de 1 à 4,

n est un nombre de 0 à 3,

$R^3$ est un groupe alkyle en $C_1$ à $C_6$.

caractérisé en ce que

a) on acyle des esters d'aminoacides de formule (VI)

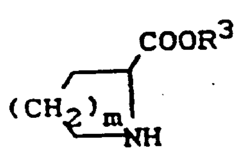

(VI)

dans laquelle

$R^3$ est un groupe alkyle et

m représente le nombre 1 ou 2,

avec des halogénures d'acides phénylacétiques de formule (VII)

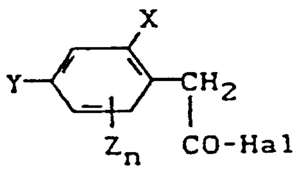

(VII)

dans laquelle
X, Y, Z et n ont la définition indiquée ci-dessus et
Hal représente le chlore ou le brome,
ou bien
b) on estérifie des acylaminoacides de formule (IIa),

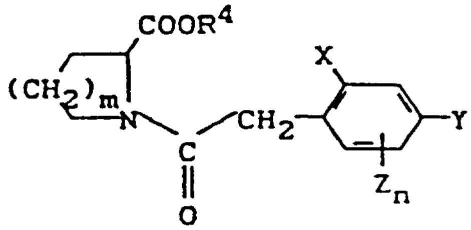

(IIa)

dans laquelle
X, Y, Z, m et n ont la définition indiquée ci-dessus et
$R^4$ représente l'hydrogène.